# EUROPEAN PATENT APPLICATION

(11) **EP 4 722 196 A1**
(43) Date of publication of application: **08.04.2026**
(21) Application number: 23938835.8
(22) Date of filing: 31.05.2023
(51) Int. Cl.: C07D 217/26, A61K 31/472, A61K 31/4725, A61P 31/14

(54) **CRYSTAL OF VIRAL PROTEASE INHIBITOR AND USE**

(71) Applicant: Westlake Pharmaceuticals (Hangzhou) Co., Ltd., Zhejiang 310024 (CN)
(72) Inventor: LIANG, Dongdong, Hangzhou, Zhejiang 310024 (CN); WU, Qi, Hangzhou, Zhejiang 310024 (CN)
(74) Representative: Ghirardi, Valeria
(86) International application number: PCT/CN2023/097358
(87) International publication number: WO 2024/243841

(57) **Abstract**

A crystal of a viral protease inhibitor and a use. The present invention specifically relates to a crystal form of a compound 1 or a pharmaceutically acceptable salt thereof and a use thereof. The present invention also relates to a preparation method for the crystal form and a composition and pharmaceutical composition comprising the crystal. The crystal form is stable, has high yield, involves mild crystallization conditions, is suitable for industrial production, and can well meet the requirements of the pharmaceutical industry.

## Description

### Technical Field

The present invention relates to crystals of anti-coronavirus protease inhibitor compounds and their preparation methods, as well as compositions or pharmaceutical compositions containing the crystals and their uses. The invention also relates to crystalline salts of the protease inhibitor compounds, including hydrochloride, fumarate, hippurate, malonate, methanesulfonate, benzenesulfonate, and p-toluenesulfonate, as well as the preparation methods of the aforementioned salts, and compositions or pharmaceutical compositions containing the above crystalline salts and their uses.

### Technical Background

Over 70% of the coronavirus genome sequence encodes 16 non-structural proteins (nsps), designated as nsp1 to nsp16. These 16 nsps are translated into two long polypeptide chains, ppla and pplab, from which individual nsps are generated through proteolysis. The hydrolysis is catalyzed by two proteases among the 16 nsps-nsp3 and nsp5. Nsp5, also known as 3CLpro, catalyzes the hydrolysis of peptide bonds linking nsp4 to nsp16. In the catalytic center of 3CLpro, there exists a catalytic dyad composed of His41 and Cys 145. The working mechanism and protein sequence of the catalytic domain of 3CLpro are conserved among different coronaviruses. The sequence identity between the 3CLpro of SARS-CoV-2 and the 3CLpro of SARS-CoV reaches 96%. Inhibiting the protease activity of 3CLpro will block the release of nsp4 to nsp 16, which are essential for coronavirus replication. Therefore, 3CLpro is a crucial target for the development of anti-coronavirus drugs.

WO2022150962A1 discloses compounds useful as inhibitors of coronavirus 3CLpro, including Compound 1 described in the present invention. The content disclosed in this patent document is incorporated in its entirety into the present application by reference. The compounds disclosed therein bind non-covalently into the catalytic pocket of 3CLpro, and competitively inhibit the binding of 3CLpro substrates thereto, thereby reducing or inhibiting the activity of 3CLpro. Furthermore, these compounds can reduce or inhibit coronavirus replication.

The sequences related to the catalytic pocket of coronavirus 3CLpro are highly conserved, and the recognized substrates also exhibit high conservation. Thus, the compounds can be pan-inhibitors of coronavirus 3CLpro, exerting therapeutic effects on infections caused by viruses of the Coronaviridae family including SARS-CoV-2, SARS-CoV, MERS-CoV, etc.

Those skilled in the art understand that the crystal form or salt form of a drug, as well as the crystal form of the salt, have a significant impact on various pharmaceutical properties, including solubility, hygroscopicity, solid-state stability, and bioavailability. Therefore, there is a need for crystal forms and/or salt forms of pharmaceutical compounds that are suitable for drug development, preparation, storage, and use.

### Disclosure of the Invention

During the research and development experiments, the inventors of the present invention have surprisingly found that the crystalline Compound 1 and certain pharmaceutically acceptable salts thereof exhibit properties particularly suitable for drug development. Therefore, an object of the present invention is to provide crystal forms of Compound 1 or its pharmaceutically acceptable salts that are suitable for drug development, pharmaceutical compositions containing the crystal forms, and medical uses of the crystal forms.

The name of Compound 1 described in the present invention is: N-((1S,2R)-2-((4-bromo-2-(methylcarbamoyl)-6-nitrophenyl)amino)cyclohexyl)isoquinoline-4-carboxamide, and its structure is as follows:

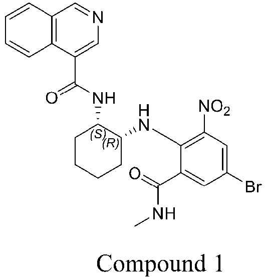

The present invention can be described from different aspects. The inventions described in these aspects and any of their embodiments are independent of yet interconnected with one another, collectively constituting the content of the present invention.

In a first aspect, the present invention provides crystal forms of Compound 1 or a pharmaceutically acceptable salt thereof.

In some embodiments, the present invention provides crystals of Compound 1 in free form.

In some embodiments, the present invention provides crystals of a pharmaceutically acceptable salt of Compound 1, wherein the salt is selected from the hydrochloride, fumarate, hippurate, malonate, methanesulfonate, benzenesulfonate, and p-toluenesulfonate of Compound 1.

In a second aspect, the present invention provides a composition comprising the crystal form according to any embodiment of the first aspect of the present invention, wherein the crystal form accounts for more than 50%, preferably more than 75%, more preferably more than 90%, and most preferably more than 95% of the mass of the composition.

In a third aspect, the present invention provides a pharmaceutical composition comprising the crystal form according to any embodiment of the first aspect of the present invention and at least one pharmaceutically acceptable carrier.

In a fourth aspect, the present invention provides the use of the crystal form according to any embodiment of the first aspect of the present invention in the preparation of a medicament for treating diseases caused by coronaviruses or symptoms thereof.

In a fifth aspect, the present invention provides the crystal form according to any embodiment of the first aspect of the present invention, the composition according to the second aspect of the present invention, or the pharmaceutical composition according to the third aspect of the present invention, for use as a medicament.

In a sixth aspect, the present invention provides the crystal form according to any embodiment of the first aspect of the present invention, the composition according to the second aspect of the present invention, or the pharmaceutical composition according to the third aspect of the present invention, for use in treating diseases caused by coronaviruses or symptoms thereof.

In a seventh aspect, the present invention provides a method for treating diseases caused by coronaviruses or symptoms thereof in a subject, comprising the step of administering a therapeutically effective amount of the crystal form according to any embodiment of the first aspect of the present invention to the subject.

It has been demonstrated by the experiments that the crystal forms of Compound 1 or its pharmaceutically acceptable salts provided by the present invention exhibit excellent performance in one or more of solubility, hygroscopicity, stability and the others, and possess favorable pharmaceutical properties and exhibit highly promising drugability.

The crystal forms of Compound 1 or its pharmaceutically acceptable salts provided by the present invention have high yield and can be prepared under mild crystallization conditions. They are suitable for industrial production and can well meet the needs of the pharmaceutical industry.

### Description of the Figures

Figure 1A is the XRPD pattern of crystal form A of Compound 1 in free form
Figure 1B is the TGA curve of crystal form A of Compound 1 in free form
Figure 1C is the DSC curve of crystal form A of Compound 1 in free form
Figure 2A is the XRPD pattern of crystal form B of Compound 1 in free form
Figure 2B is the TGA curve of crystal form B of Compound 1 in free form
Figure 2C is the DSC curve of crystal form B of Compound 1 in free form
Figure 3 is the XRPD pattern of crystal form C of Compound 1 in free form
Figure 4A is the XRPD pattern of crystal form A of Compound 1 hydrochloride
Figure 4B is the TGA curve of crystal form A of Compound 1 hydrochloride
Figure 4C is the DSC curve of crystal form A of Compound 1 hydrochloride
Figure 5A is the XRPD pattern of crystal form B of Compound 1 hydrochloride
Figure 5B is the TGA curve of crystal form B of Compound 1 hydrochloride
Figure 5C is the DSC curve of crystal form B of Compound 1 hydrochloride
Figure 6A is the XRPD pattern of crystal form A of Compound 1 fumarate salt
Figure 6B is the TGA/DSC curve of crystal form A of Compound 1 fumarate salt
Figure 7A is the XRPD pattern of crystal form A of Compound 1 hippurate salt
Figure 7B is the TGA curve of crystal form A of Compound 1 hippurate salt
Figure 7C is the DSC curve of crystal form A of Compound 1 hippurate salt
Figure 8A is the XRPD pattern of crystal form A of Compound 1 malonate salt
Figure 8B is the TGA curve of crystal form A of Compound 1 malonate salt
Figure 8C is the DSC curve of crystal form A of Compound 1 malonate salt
Figure 9A is the XRPD pattern of crystal form A of Compound 1 methanesulfonate salt
Figure 9B is the TGA curve of crystal form A of Compound 1 methanesulfonate salt
Figure 9C is the DSC curve of crystal form A of Compound 1 methanesulfonate salt
Figure 10A is the XRPD pattern of crystal form A of Compound 1 benzenesulfonate salt
Figure 10B is the TGA curve of crystal form A of Compound 1 benzenesulfonate salt
Figure 10C is the DSC curve of crystal form A of Compound 1 benzenesulfonate salt
Figure 11A is the XRPD pattern of crystal form B of Compound 1 benzenesulfonate salt
Figure 11B is the TGA curve of crystal form B of Compound 1 benzenesulfonate salt
Figure 11C is the DSC curve of crystal form B of Compound 1 benzenesulfonate salt
Figure 12A is the XRPD pattern of crystal form A of Compound 1 p-toluenesulfonate salt
Figure 12B is the TGA curve of crystal form A of Compound 1 p-toluenesulfonate salt
Figure 12C is the DSC curve of crystal form A of Compound 1 p-toluenesulfonate salt
Figure 13A is the XRPD pattern of crystal form B of Compound 1 p-toluenesulfonate salt
Figure 13B is the TGA curve of crystal form B of Compound 1 p-toluenesulfonate salt
Figure 13C is the DSC curve of crystal form B of Compound 1 p-toluenesulfonate salt

### Detailed Description

### 1. Definition

Unless otherwise specified, the following terms and phrases used in the present invention have the following meanings:
The term "coronavirus", as used herein, refers to a group of related RNA viruses that cause diseases in mammals and birds, such as respiratory infectious diseases, typically in humans and birds, which can range from mild to fatal diarrhea in cattle and pigs, as well as hepatitis and encephalomyelitis in mice. Coronaviruses are enveloped viruses with a positive-sense single-stranded RNA genome and a helically symmetric nucleocapsid. They have characteristic rod-like spikes protruding from the surface, which in electron micrographs evoke a reminiscent of the shape of the corona of the sun, from which the name of coronavirus comes. The lethal coronavirus category includes coronaviruses causing diseases such as SARS (severe acute respiratory syndrome), MERS (middle east respiratory syndrome) and COVID 19 (contagious disease caused by severe acute respiratory syndrome coronavirus 2).

The terms "pharmaceutically acceptable" and "medicinally acceptable", as used herein, refer to components that, within the scope of sound medical judgment, are suitable for contact with the tissues of humans and other mammals without excessive toxicity, irritation, allergic reactions, etc., and are commensurate with a reasonable benefit/risk ratio. A "pharmaceutically acceptable salt" refers to any salt that is non-toxic to the recipient and can directly or indirectly provide the compound of the present invention after administration to the recipient. Suitable pharmaceutically acceptable salts are, for example, those disclosed by S. M. Berge et al. in J. Pharmaceutical Sciences, 1977, 66, pp. 1-19. For instance, pharmaceutically acceptable salts of Compound 1 include, but are not limited to, sulfate, pyrosulfate, bisulfate, sulfite, bisulfite, phosphate, monohydrogen phosphate, dihydrogen phosphate, metaphosphate, pyrophosphate, chloride, bromide, iodide, acetate, propionate, caprate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylenesulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2-sulfonate, mandelate, and other salts of Compound 1.

The terms "subject," "patient," or "recipient", as used herein, refer to animals susceptible to coronavirus infection, preferably mammals, and particularly humans.

The term "therapeutically effective amount", as used herein, refers to the amount of Compound 1 or a pharmaceutically acceptable salt thereof required to produce the intended therapeutic effect (e.g., improvement of diseases and/or symptoms caused by coronaviruses, reduction of the severity of diseases and/or symptoms caused by coronaviruses, and/or slowing the progression of diseases and/or symptoms caused by coronaviruses). The precise amount as the therapeutically effective amount will depend on the purpose of treatment and can be determined by those skilled in the art using known techniques.

The term "treatment", as used herein, includes, but is not limited to, the following: complete or partial alleviation, or cure of diseases caused by coronaviruses, and reduction of the risk of diseases caused by coronaviruses and/or symptoms thereof. The improvement or reduction in the severity of any of these symptoms can be evaluated according to methods and techniques known in the art.

The term "room temperature", as used herein, means ambient temperature, for example, 10-40°C, preferably 15-35°C, more preferably 20-30°C, such as around 25°C.

The terms "about" and "approximately", as used herein, when used in conjunction with numbers such as percentages, include the specified numbers as well as numerical ranges recognized by those skilled in the art (e.g., ±10%; preferably, ±5%; more preferably, ±2%; most preferably, ±1%).

The phrases "having characteristic diffraction peaks approximately at the following 20 angles ..." and "the XRPD pattern having diffraction peaks at 2θ angles of approximately ...", as used herein, mean that the X-ray powder diffraction (XRPD) pattern has diffraction peaks within the range of ±0.5°, preferably ±0.2°, more preferably ±0.1° of the indicated 2θ angles.

The phrase "at ...°C", as used herein, means a range around that temperature, for example, an endothermic signal "at 65.3°C" means an endothermic signal within the range of 65.3°C±1°C, preferably ±0.5°C, more preferably ±0.2°C.

### 2. Crystals of the present invention

In some embodiments, the present invention provides crystal form A of Compound 1 in free form, which exhibits a X-ray powder diffraction (XRPD) pattern having characteristic diffraction peaks approximately at the following 20 angles: 9.32, 10.39, 13.18, 19.76, and 23.84.

In some embodiments, the XRPD pattern of crystal form A of Compound 1 in free form has characteristic diffraction peaks approximately at the following 20 angles: 9.32, 10.39, 13.18, 16.11, 16.78, 19.23, 19.76, 20.30, 20.84, 23.38, and 23.84.

In some embodiments, the XRPD pattern of crystal form A of Compound 1 in free form has characteristic diffraction peaks approximately at the following 20 angles: 6.51, 9.32, 10.39, 11.30, 13.18, 14.67, 16.11, 16.78, 17.98, 19.23, 19.76, 20.30, 20.84, 21.21, 21.95, 23.38, 23.84, 24.64, 25.95, 27.30, 28.08, 30.23, 31.44, 33.93, 36.56, and 38.72.

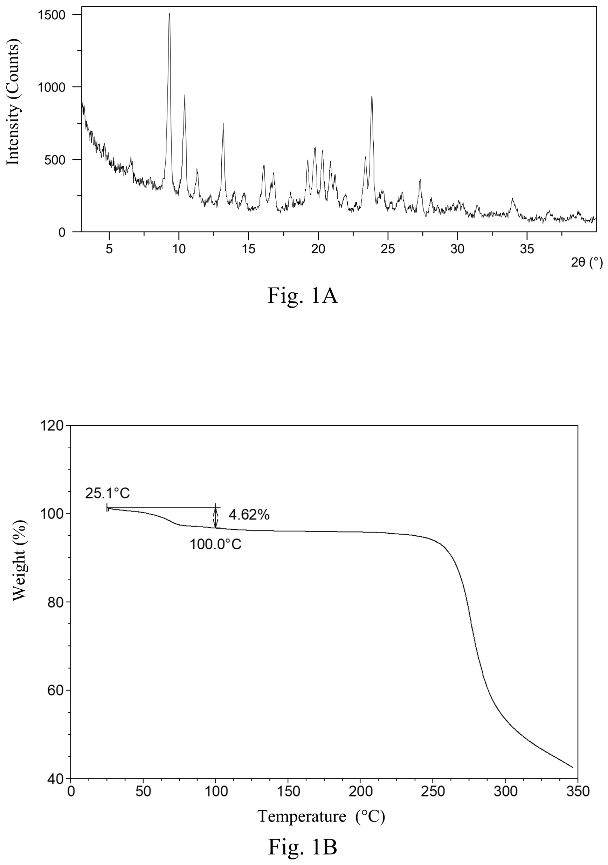

In some embodiments, the XRPD pattern of crystal form A of Compound 1 in free form is substantially as shown in Figure 1A.

In some embodiments, the thermogravimetric analysis (TGA) result of crystal form A of Compound 1 in free form is substantially as shown in Figure 1B, with a stepwise weight loss of about 4.62% when the sample is heated to about 100°C.

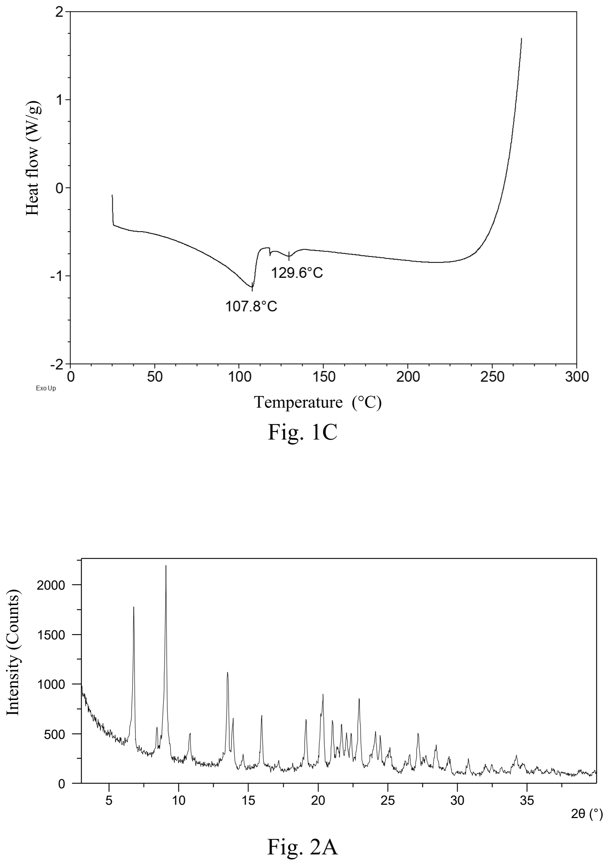

In some embodiments, the differential scanning calorimetry (DSC) result of crystal form A of Compound 1 in free form is substantially as shown in Figure 1C, with two endothermic signals at about 107.8°C and 129.6°C (peak temperatures).

In some embodiments, the present invention provides crystal form B of Compound 1 in free form, which exhibits an XRPD pattern having characteristic diffraction peaks approximately at the following 2θ angles: 6.76, 9.07, 13.50, 20.33, and 22.95.

In some embodiments, the XRPD pattern of crystal form B of Compound 1 in free form has characteristic diffraction peaks approximately at the following 20 angles: 6.76, 9.07, 13.50, 13.89, 15.94, 19.10, 20.16, 20.33, 21.03, 21.68, 22.36, 22.95, 24.10, and 27.14.

In some embodiments, the XRPD pattern of crystal form B of Compound 1 in free form has characteristic diffraction peaks approximately at the following 2θ angles: 6.76, 8.40, 9.07, 10.79, 13.50, 13.89, 14.61, 15.94, 17.10, 19.10, 20.16, 20.33, 21.03, 21.31, 21.68, 22.00, 22.36, 22.95, 24.10, 24.46, 25.13, 26.53, 27.14, 27.75, 28.46, 29.39, 30.78, 31.98, 34.20, 35.70, and 38.80.

In some embodiments, the XRPD pattern of crystal form B of Compound 1 in free form is substantially as shown in Figure 2A.

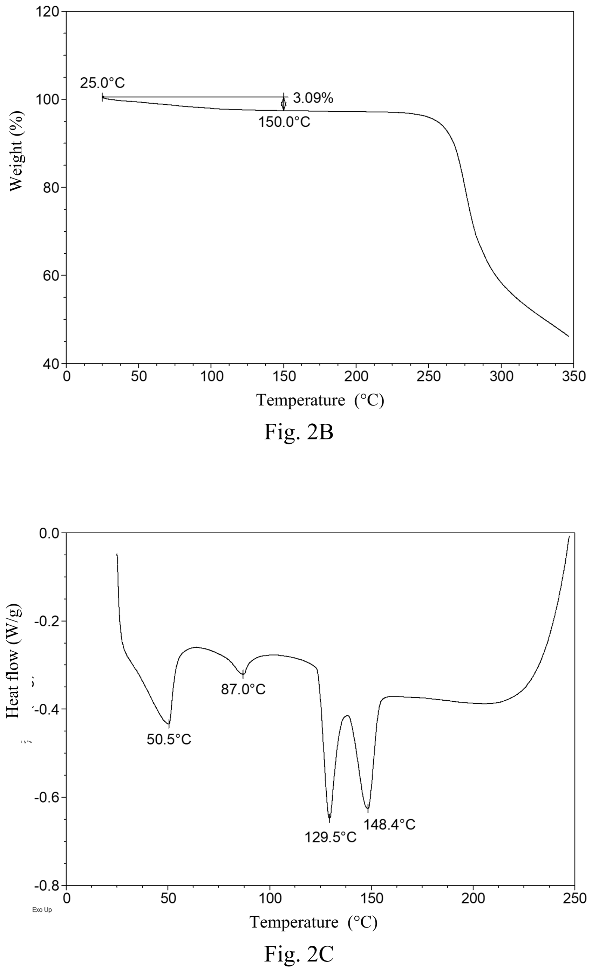

In some embodiments, the TGA result of crystal form B of Compound 1 in free form is substantially as shown in Figure 2B, with a weight loss of about 3.09% when the sample is heated to about 150°C.

In some embodiments, the DSC characterization result of crystal form B of Compound 1 in free form is substantially as shown in Figure 2C, with four endothermic signals at about 50.5°C, 87.0°C, 129.5°C, and 148.4°C (peak temperatures). When crystal form B of Compound 1 in free form is heated to about 128°C and then cooled to room temperature, XRPD results show that the crystal form remains unchanged before and after heating; the sample melts after being heated to about 155°C.

In some embodiments, the present invention provides crystal form C of Compound 1 in free form, which exhibits an XRPD pattern having characteristic diffraction peaks approximately at the following 2θ angles: 8.64, 9.23, and 12.99.

In some embodiments, the XRPD pattern of crystal form C of Compound 1 in free form has characteristic diffraction peaks approximately at the following 2θ angles: 8.64, 9.23, 12.99, and 20.29.

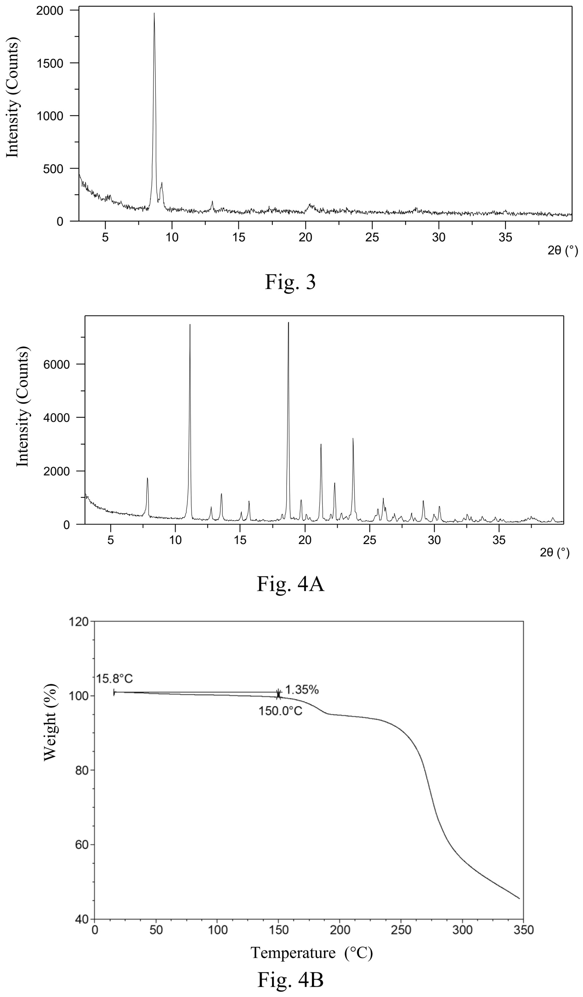

In some embodiments, the XRPD result of crystal form C of Compound 1 in free form is substantially as shown in Figure 3.

In some embodiments, the present invention provides crystal form A of Compound 1 hydrochloride, which exhibits an XRPD pattern having characteristic diffraction peaks approximately at the following 2θ angles: 7.84, 11.11, 18.71, 21.24, and 23.72.

In some embodiments, the XRPD pattern of crystal form A of Compound 1 hydrochloride has characteristic diffraction peaks approximately at the following 20 angles: 7.84, 11.11, 13.54, 18.71, 19.70, 21.24, 22.29, 23.72, and 26.03.

In some embodiments, the XRPD pattern of crystal form A of Compound 1 hydrochloride has characteristic diffraction peaks approximately at the following 20 angles: 7.84, 11.11, 12.74, 13.54, 15.07, 15.67, 18.22, 18.71, 19.70, 20.10, 21.24, 21.99, 22.29, 22.81, 23.72, 25.63, 26.03, 26.23, 26.91, 27.42, 28.22, 29.12, 29.94, 30.37, 32.50, 33.68, 34.70, 37.64, and 39.12.

In some embodiments, the XRPD result of crystal form A of Compound 1 hydrochloride is substantially as shown in Figure 4A.

In some embodiments, the TGA result of crystal form A of Compound 1 hydrochloride is substantially as shown in Figure 4B, with a weight loss of about 1.35% when the sample is heated to about 150°C.

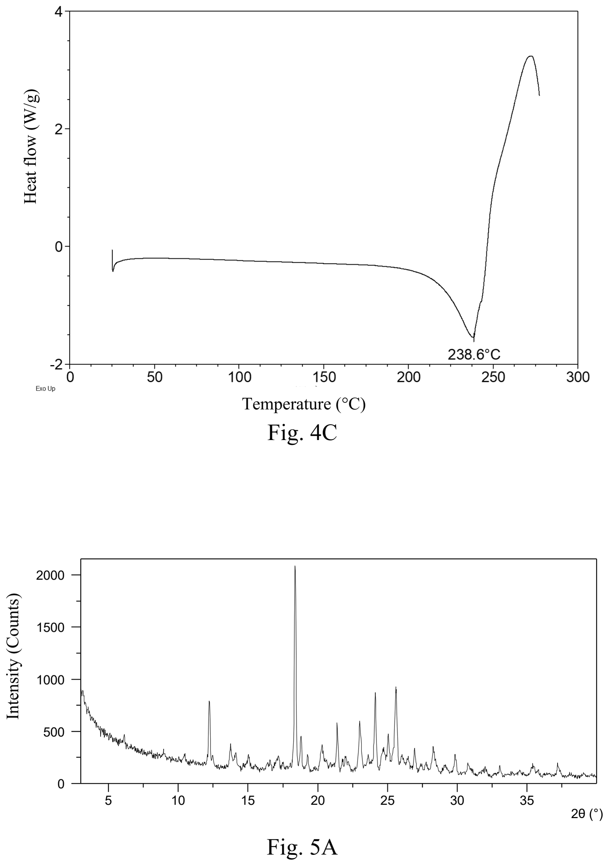

In some embodiments, the DSC result of crystal form A of Compound 1 hydrochloride is substantially as shown in Figure 4C, with one endothermic signal at about 238.6°C (peak temperature).

In some embodiments, the molar ratio of acid to base of crystal form A of Compound 1 hydrochloride is 1:0.5-2, preferably 1:0.9-1.1, and more preferably 1:1.

In some embodiments, the present invention provides crystal form B of Compound 1 hydrochloride, which exhibits an XRPD pattern having characteristic diffraction peaks approximately at the following 20 angles: 12.22, 18.37, 22.99, 24.11, and 25.59.

In some embodiments, the XRPD pattern of crystal form B of Compound 1 hydrochloride has characteristic diffraction peaks approximately at the following 20 angles: 12.22, 13.72, 18.37, 18.79, 20.28, 21.39, 22.99, 24.11, 25.03, 25.59, 26.92, and 28.27.

In some embodiments, the XRPD pattern of crystal form B of Compound 1 hydrochloride has characteristic diffraction peaks approximately at the following 20 angles: 2.22, 13.72, 14.10, 15.04, 17.15, 18.37, 18.79, 19.26, 20.28, 20.93, 21.39, 21.98, 22.99, 24.11, 24.64, 25.03, 25.59, 26.03, 26.45, 26.92, 28.27, 29.83, 30.77, 31.96, 33.05, 35.43, and 37.20.

In some embodiments, the XRPD result of crystal form B of Compound 1 hydrochloride is substantially as shown in Figure 5A.

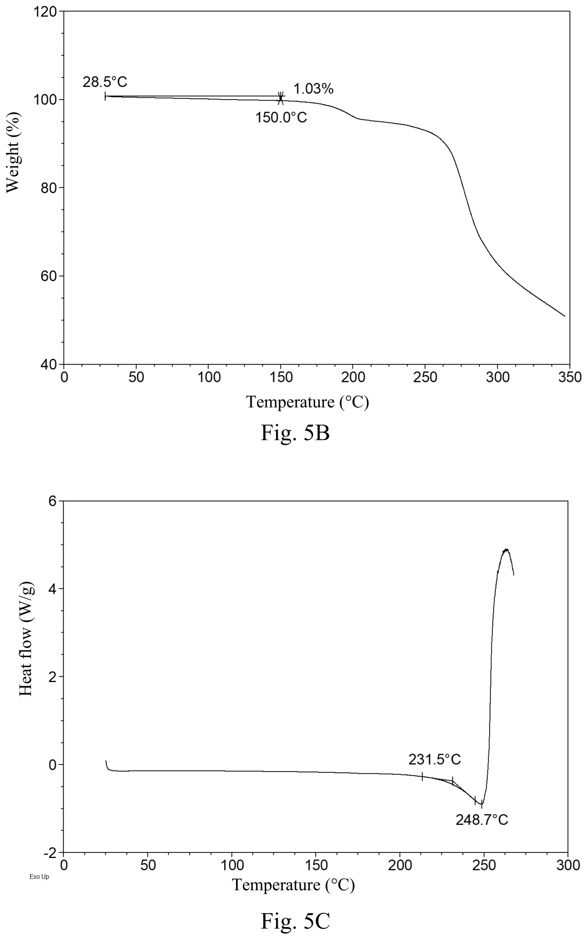

In some embodiments, the TGA result of crystal form B of Compound 1 hydrochloride is substantially as shown in Figure 5B, with a weight loss of about 1.03% when the sample is heated to about 150°C.

In some embodiments, the DSC result of crystal form B of Compound 1 hydrochloride is substantially as shown in Figure 5C, with one endothermic signal at about 248.7°C (peak temperature).

In some embodiments, the molar ratio of acid to base of crystal form B of Compound 1 hydrochloride is 1:0.5-2, preferably 1:0.9-1.1, and more preferably 1:1.

In some embodiments, the present invention provides crystal form A of Compound 1 fumarate salt, which exhibits an XRPD pattern having characteristic diffraction peaks approximately at the following 2θ angles: 9.19, 10.92, 18.66, 19.48, and 20.01.

In some embodiments, the XRPD pattern of crystal form A of Compound 1 fumarate salt has characteristic diffraction peaks approximately at the following 20 angles: 9.19, 10.92, 11.97, 18.66, 18.90, 19.48, 20.01, 20.21, 23.28, 23.49, and 23.68.

In some embodiments, the XRPD pattern of crystal form A of Compound 1 fumarate salt has characteristic diffraction peaks approximately at the following 20 angles: 5.91, 9.19, 10.00, 10.92, 11.97, 12.76, 14.60, 15.94, 17.35, 18.66, 18.90, 19.48, 20.01, 20.21, 20.49, 21.21, 21.94, 23.28, 23.49, 23.68, 24.34, 24.95, 25.70, 26.21, 27.30, 27.98, 28.43, 29.21, 29.72, 30.41, 31.21, 32.19, 33.44, 34.94, and 36.77.

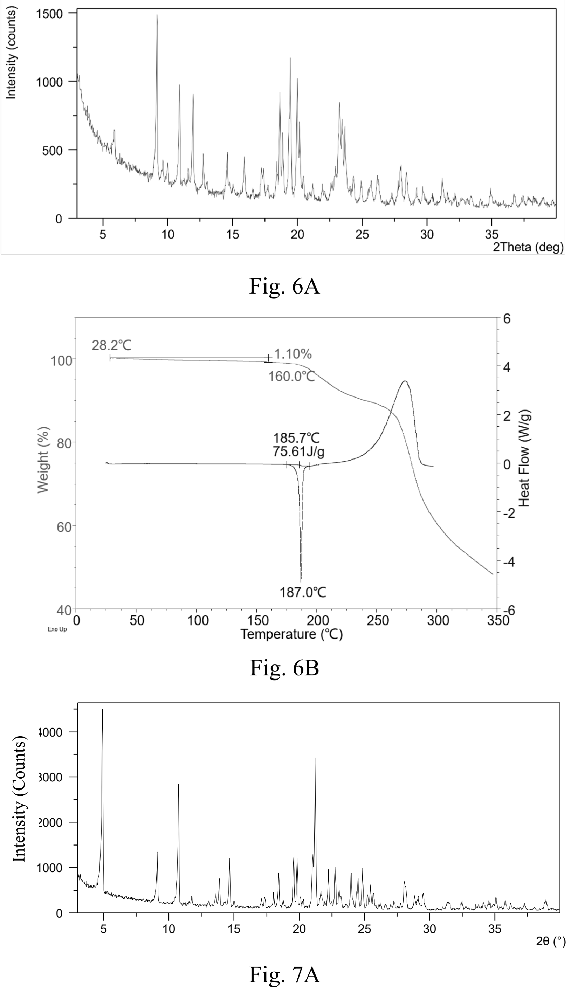

In some embodiments, the XRPD result of crystal form A of Compound 1 fumarate salt is substantially as shown in Figure 6A.

In some embodiments, the TGA/DSC result of crystal form A of Compound 1 fumarate salt is substantially as shown in Figure 6B, with a weight loss of about 1.10% when the sample is heated to about 160°C, and one sharp endothermic peak at about 187°C (peak temperature).

In some embodiments, the molar ratio of acid to base of crystal form A of Compound 1 fumarate salt is 1:0.5-2, preferably about 1:0.9 or about 1:2.

In some embodiments, the present invention provides crystal form A of Compound 1 hippurate salt, which exhibits an XRPD pattern having characteristic diffraction peaks approximately at the following 2θ angles: 4.90, 9.09, 10.73, 19.56, and 21.21.

In some embodiments, the XRPD pattern of crystal form A of Compound 1 hippurate salt has characteristic diffraction peaks approximately at the following 20 angles: 4.90, 9.09, 10.73, 14.64, 19.56, 19.82, 21.01, 21.21, 22.74, 23.96, and 24.84.

In some embodiments, the XRPD pattern of crystal form A of Compound 1 hippurate salt has characteristic diffraction peaks approximately at the following 20 angles: 4.90, 9.09, 10.73, 11.74, 13.06, 13.59, 13.87, 14.64, 14.99, 17.10, 17.34, 18.02, 18.41, 18.74, 19.56, 19.82, 20.08, 20.28, 21.01, 21.21, 21.65, 22.22, 22.74, 23.09, 23.96, 24.50, 24.84, 25.22, 25.44, 25.67, 26.17, 26.57, 27.25, 28.03, 28.81, 29.09, 29.47, 31.46, 32.46, 34.09, 35.06, 35.77, 36.18, 37.25, and 38.90.

In some embodiments, the XRPD result of crystal form A of Compound 1 hippurate salt is substantially as shown in Figure 7A.

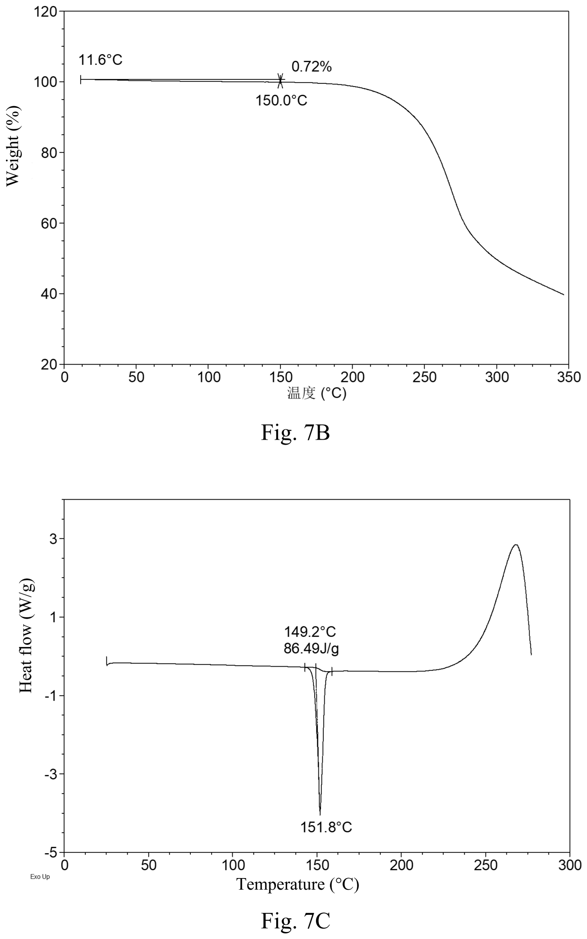

In some embodiments, the TGA result of crystal form A of Compound 1 hippurate salt is substantially as shown in Figure 7B, with a weight loss of about 0.72% when the sample is heated to about 150°C.

In some embodiments, the DSC result of crystal form A of Compound 1 hippurate salt is substantially as shown in Figure 7C, with one sharp endothermic peak at about 151.8°C (peak temperature).

In some embodiments, the molar ratio of acid to base of crystal form A of Compound 1 hippurate salt is 1:0.5-2, preferably 1:0.9-1.1, and more preferably 1:1.

In some embodiments, the present invention provides crystal form A of Compound 1 malonate salt, which exhibits an XRPD pattern having characteristic diffraction peaks approximately at the following 2θ angles: 5.36, 8.37, and 21.39.

In some embodiments, the XRPD pattern of crystal form A of Compound 1 malonate salt has characteristic diffraction peaks approximately at the following 20 angles: 5.36, 8.37, 20.49, 21.39, and 23.75.

In some embodiments, the XRPD pattern of crystal form A of Compound 1 malonate salt has characteristic diffraction peaks approximately at the following 20 angles: 5.36, 8.37, 9.90, 11.54, 16.99, 20.49, 21.39, 22.50, 23.75, 25.09, and 27.35.

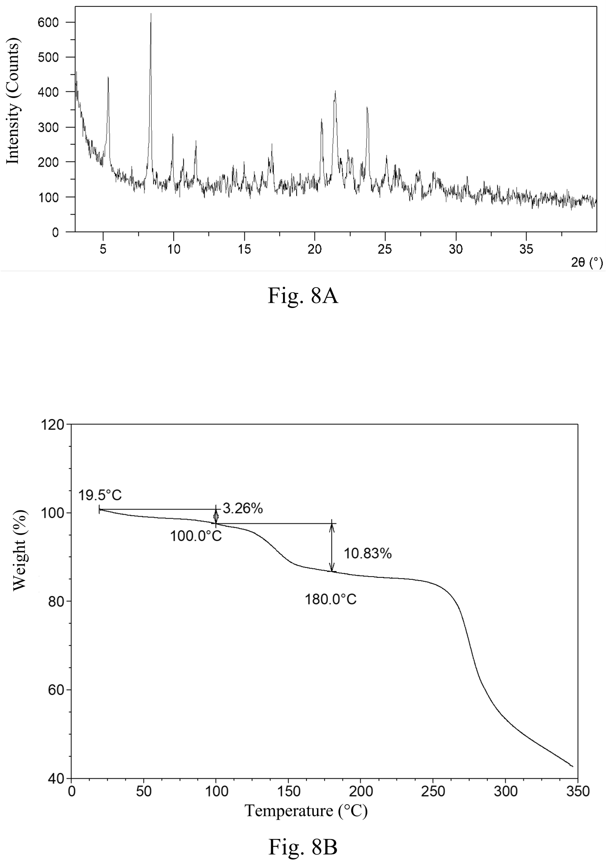

In some embodiments, the XRPD result of crystal form A of Compound 1 malonate salt is substantially as shown in Figure 8A.

In some embodiments, the TGA result of crystal form A of Compound 1 malonate salt is substantially as shown in Figure 8B, with a weight loss of about 3.26% when the sample is heated to about 100°C, and about 10.83% when further heated to about 180°C.

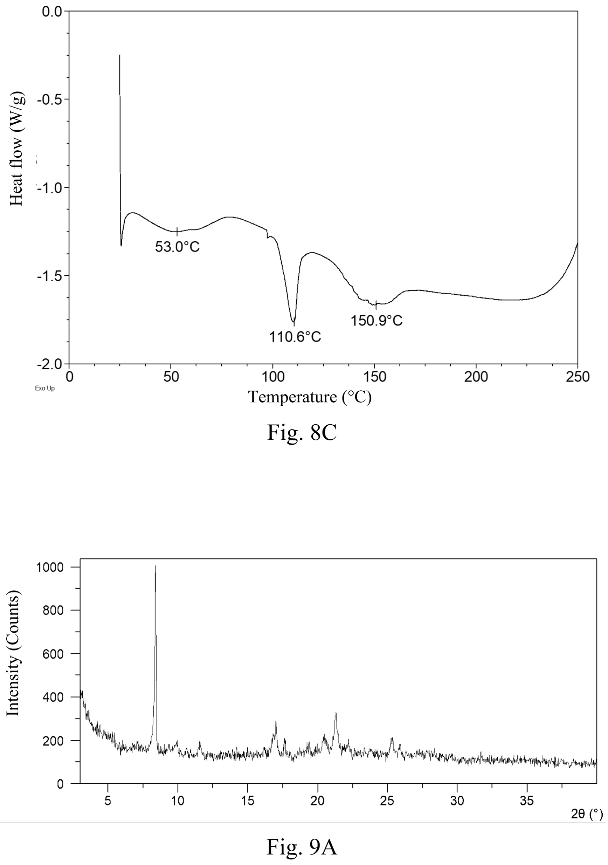

In some embodiments, the DSC result of crystal form A of Compound 1 malonate salt is substantially as shown in Figure 8C, with three endothermic signals at about 53.0°C, 110.6°C, and 150.9°C (peak temperatures).

In some embodiments, the molar ratio of acid to base of crystal form A of Compound 1 malonate salt is 1:0.5-2, preferably 1:0.9-1.1, and more preferably 1:1.

In some embodiments, the present invention provides crystal form A of Compound 1 methanesulfonate salt, which exhibits an XRPD pattern having characteristic diffraction peaks approximately at the following 20 angles: 8.40, 16.95, and 21.30.

In some embodiments, the XRPD pattern of crystal form A of Compound 1 methanesulfonate salt has characteristic diffraction peaks approximately at the following 20 angles: 8.40, 11.59, 16.95, 21.30, and 25.31.

In some embodiments, the XRPD result of crystal form A of Compound 1 methanesulfonate salt is substantially as shown in Figure 9A.

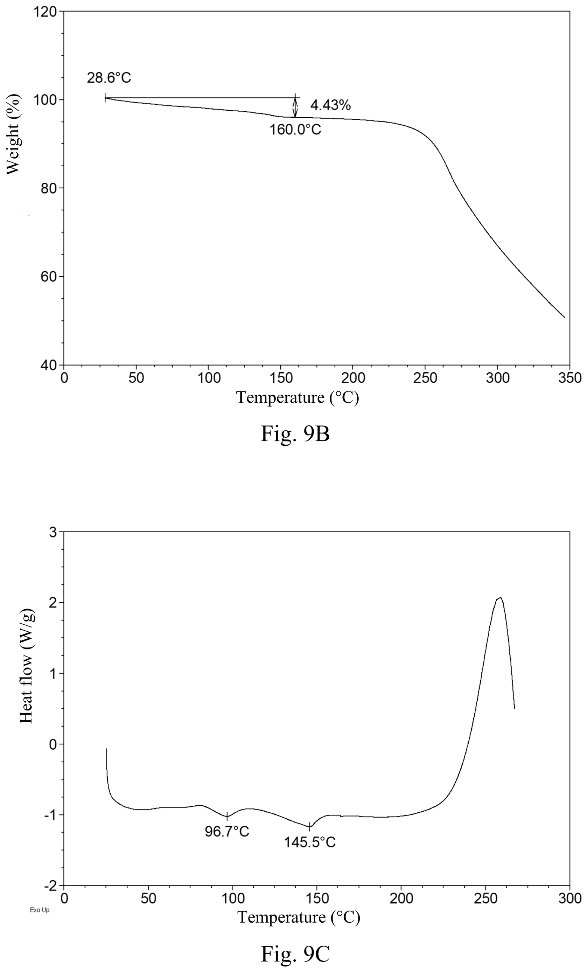

In some embodiments, the TGA result of crystal form A of Compound 1 methanesulfonate salt is substantially as shown in Figure 9B, with a stepwise weight loss of about 4.43% when the sample is heated to about 160°C.

In some embodiments, the DSC result of crystal form A of Compound 1 methanesulfonate salt is substantially as shown in Figure 9C, with two endothermic signals at about 96.7°C and 145.5°C (peak temperatures).

In some embodiments, the molar ratio of acid to base of crystal form A of Compound 1 methanesulfonate salt is 1:0.5-2, preferably 1:0.9-1.1, and more preferably 1:1.

In some embodiments, the present invention provides crystal form A of Compound 1 benzenesulfonate salt, which exhibits an XRPD pattern having characteristic diffraction peaks approximately at the following 2θ angles: 8.62, 19.42, 24.28, 25.62, and 26.00.

In some embodiments, the XRPD pattern of crystal form A of Compound 1 benzenesulfonate salt has characteristic diffraction peaks approximately at the following 20 angles: 8.62, 11.40, 17.24, 19.42, 20.69, 22.28, 23.62, 24.28, 25.62, 26.00, and 27.07.

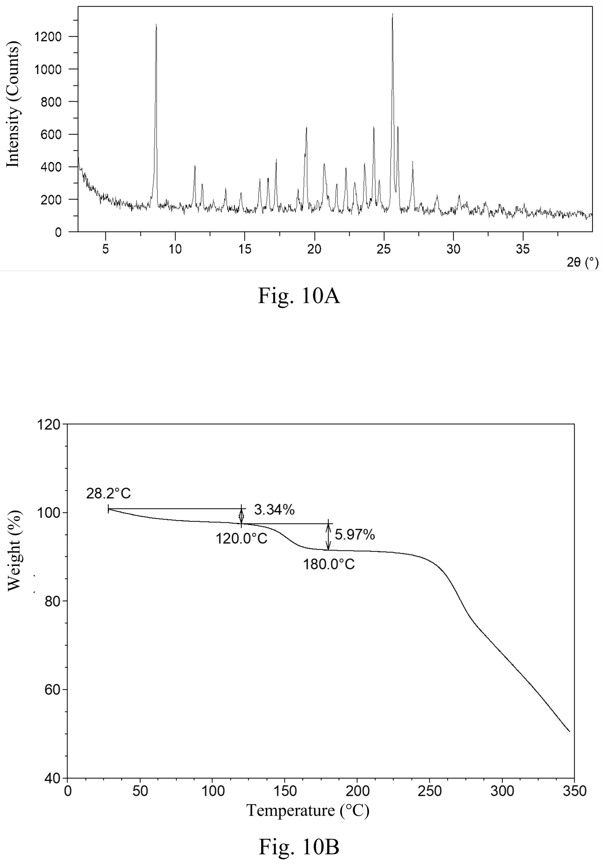

In some embodiments, the XRPD result of crystal form A of Compound 1 benzenesulfonate salt is substantially as shown in Figure 10A.

In some embodiments, the TGA result of crystal form A of Compound 1 benzenesulfonate salt is substantially as shown in Figure 10B, with a weight loss of about 3.34% when the sample is heated to about 120°C, and about 5.97% when further heated to about 180°C.

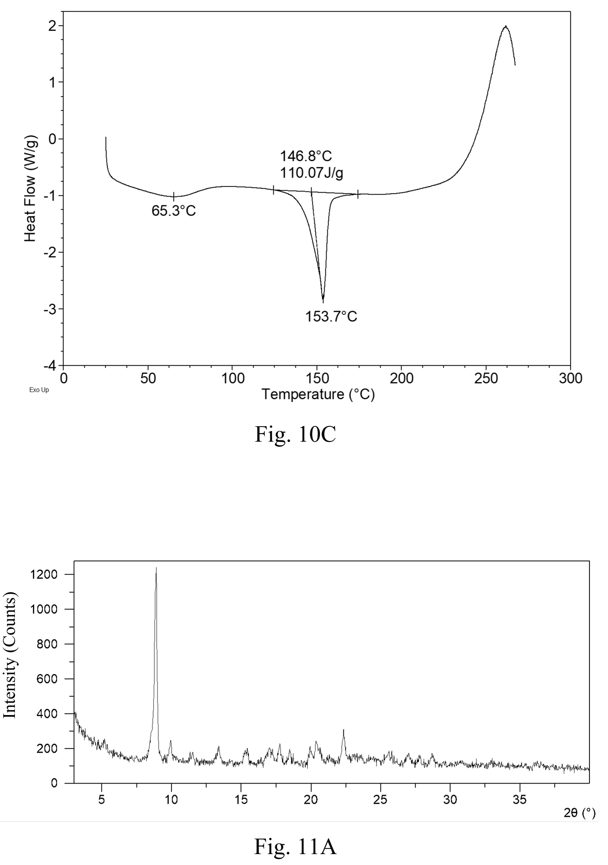

In some embodiments, the DSC result of crystal form A of Compound 1 benzenesulfonate salt is substantially as shown in Figure 10C, with two endothermic signals at about 65.3°C and 153.7°C (peak temperatures).

In some embodiments, the molar ratio of acid to base of crystal form A of Compound 1 benzenesulfonate salt is 1:0.5-2, preferably 1:0.9-1.1, and more preferably 1:1.

In some embodiments, the present invention provides crystal form B of Compound 1 benzenesulfonate salt, which exhibits an XRPD pattern having characteristic diffraction peaks approximately at the following 2θ angles: 8.89, 9.93, and 22.35.

In some embodiments, the XRPD pattern of crystal form B of Compound 1 benzenesulfonate salt has characteristic diffraction peaks approximately at the following 2θ angles: 8.89, 9.93, 17.75, 20.49, and 22.35.

In some embodiments, the XRPD pattern of crystal form B of Compound 1 benzenesulfonate salt has characteristic diffraction peaks approximately at the following 2θ angles: 8.89, 9.93, 13.36, 15.35, 17.05, 17.75, 18.49, 20.49, 22.35, 25.58, 26.99, and 28.69.

In some embodiments, the XRPD result of crystal form B of Compound 1 benzenesulfonate salt is substantially as shown in Figure 11A.

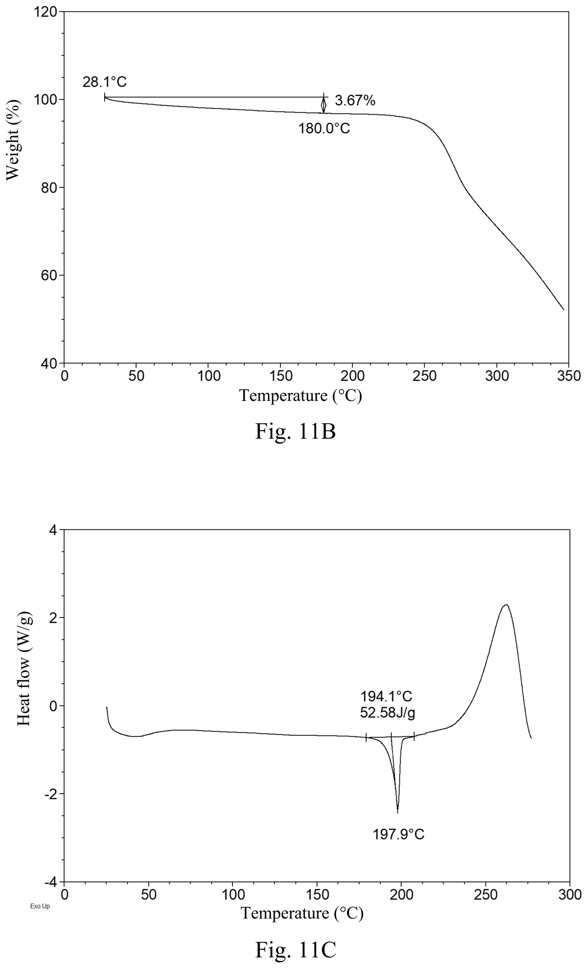

In some embodiments, the TGA result of crystal form B of Compound 1 benzenesulfonate salt is substantially as shown in Figure 11B, with a weight loss of about 3.67% when the sample is heated to about 180°C.

In some embodiments, the DSC result of crystal form B of Compound 1 benzenesulfonate salt is substantially as shown in Figure 11C, with one sharp endothermic peak at about 194.1°C (onset temperature).

In some embodiments, the molar ratio of acid to base of crystal form B of Compound 1 benzenesulfonate salt is 1:0.5-2, preferably 1:0.9-1.1, and more preferably 1:1.

In some embodiments, the present invention provides crystal form A of Compound 1 p-toluenesulfonate salt, which exhibits an XRPD pattern having characteristic diffraction peaks approximately at the following 2θ angles: 8.38, 19.21, 24.16, 25.75, and 27.09.

In some embodiments, the XRPD pattern of crystal form A of Compound 1 p-toluenesulfonate salt has characteristic diffraction peaks approximately at the following 2θ angles: 8.38, 11.42, 16.78, 19.21, 20.55, 21.73, 22.29, 24.16, 25.75, and 27.09.

In some embodiments, the XRPD pattern of crystal form A of Compound 1 p-toluenesulfonate salt has characteristic diffraction peaks approximately at the following 2θ angles: 8.38, 11.42, 11.93, 14.59, 16.09, 16.45, 16.78, 18.64, 19.21, 20.55, 21.73, 22.29, 24.16, 25.75, 27.09, and 30.07.

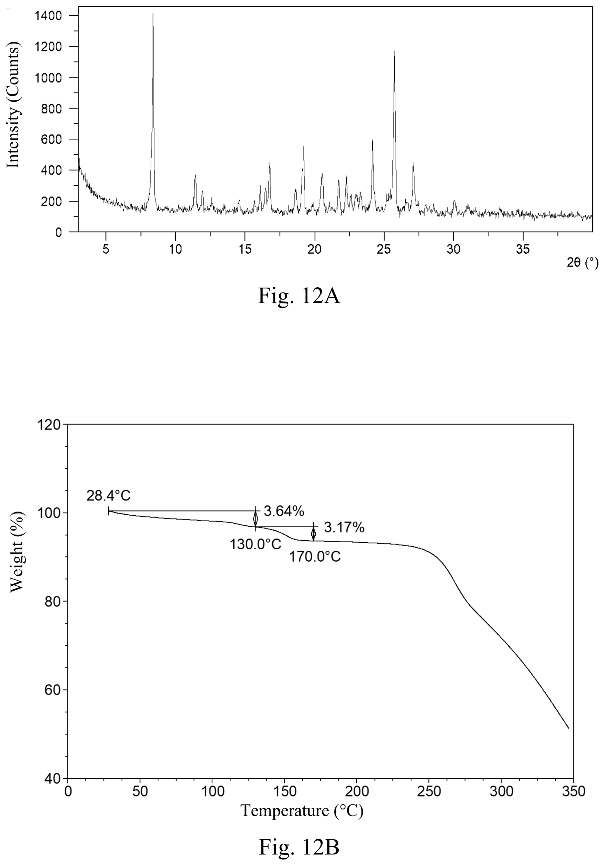

In some embodiments, the XRPD result of crystal form A of Compound 1 p-toluenesulfonate salt is substantially as shown in Figure 12A.

In some embodiments, the TGA result of crystal form A of Compound 1 p-toluenesulfonate salt is substantially as shown in Figure 12B, with a weight loss of about 3.64% when the sample is heated to about 130°C, and about 3.17% when further heated to about 170°C.

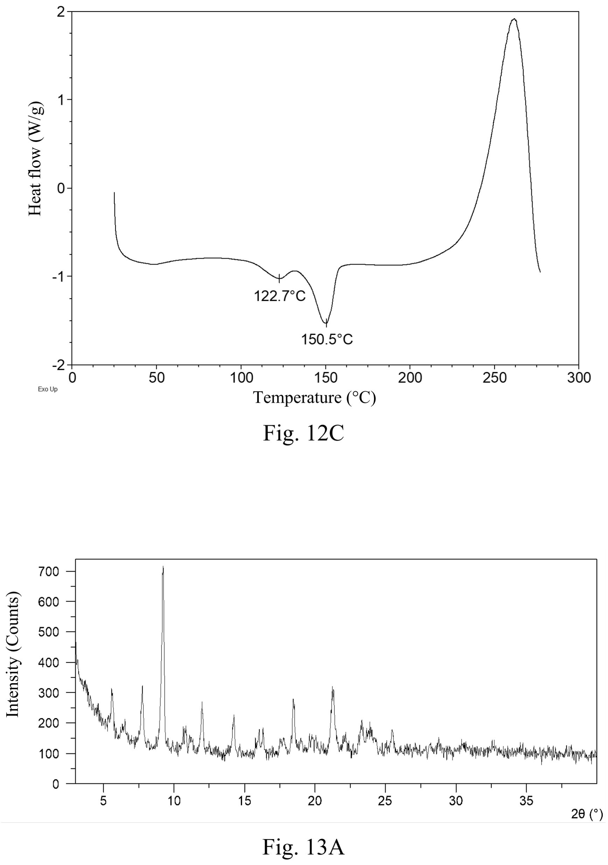

In some embodiments, the DSC result of crystal form A of Compound 1 p-toluenesulfonate salt is substantially as shown in Figure 12C, with two endothermic signals at about 122.7°C and 150.5°C (peak temperatures).

In some embodiments, the molar ratio of acid to base of crystal form A of Compound 1 p-toluenesulfonate salt is 1:0.5-2, preferably 1:0.9-1.1, and more preferably 1:1.

In some embodiments, the present invention provides crystal form B of Compound 1 p-toluenesulfonate salt, which exhibits an XRPD pattern having characteristic diffraction peaks approximately at the following 2θ angles: 7.76, 9.22, 11.99, 18.44, and 21.26.

In some embodiments, the XRPD pattern of crystal form B of Compound 1 p-toluenesulfonate salt has characteristic diffraction peaks approximately at the following 2θ angles: 5.61, 7.76, 9.22, 11.99, 14.22, 18.44, 21.26, and 23.33.

In some embodiments, the XRPD pattern of crystal form B of Compound 1 p-toluenesulfonate salt has characteristic diffraction peaks approximately at the following 2θ angles: 5.61, 7.76, 9.22, 10.88, 11.99, 14.22, 15.91, 17.69, 18.44, 21.26, and 23.33.

In some embodiments, the XRPD result of crystal form B of Compound 1 p-toluenesulfonate salt is substantially as shown in Figure 13A.

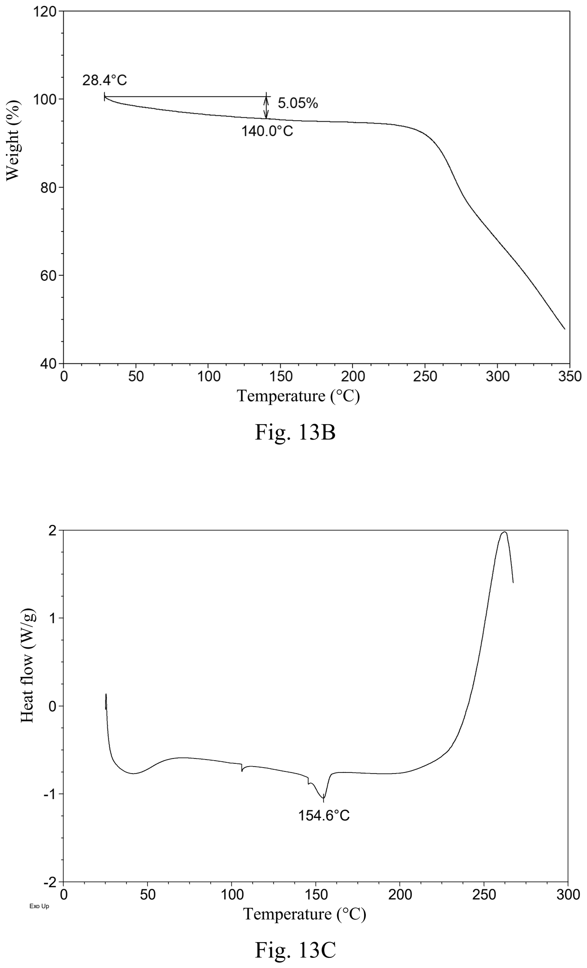

In some embodiments, the TGA result of crystal form B of Compound 1 p-toluenesulfonate salt is substantially as shown in Figure 13B, with a weight loss of about 5.05% when the sample is heated to about 140°C.

In some embodiments, the DSC result of crystal form B of Compound 1 p-toluenesulfonate salt is substantially as shown in Figure 13C, with one endothermic signal at about 154.6°C (peak temperature).

In some embodiments, the molar ratio of acid to base of crystal form A of Compound 1 p-toluenesulfonate salt is 1:0.5-2, preferably 1:0.9-1.1, and more preferably 1:1.

### 3. Preparation of the crystals of the present invention

The crystal forms according to any embodiment of the first aspect of the present invention can be prepared according to the methods disclosed in the present application or their obvious alternatives in combination with common technical knowledge and conventional experimental means in the art.

In some embodiments, crystal form A of Compound 1 in free form is obtained by stirring Compound 1 in an aqueous solution of acetonitrile at room temperature, followed by drying to yield the crystals.

Preferably, crystal form A of Compound 1 in free form is obtained by stirring an amorphous sample of Compound 1 in ACN/H₂O (1:1, v/v) at room temperature for about 2 days, then drying under vacuum overnight.

In some embodiments, crystal form B of Compound 1 in free form is obtained by stirring Compound 1 in an aqueous methanol solution at room temperature, followed by drying to yield the crystals.

Preferably, crystal form B of Compound 1 in free form is obtained by stirring an amorphous sample of Compound 1 in MeOH/H₂O (1:1, v/v) at room temperature for about 2 days, then drying under vacuum overnight.

In some embodiments, crystal form C of Compound 1 in free form is obtained by volatilizing the solvent from a solution of Compound 1 in an aqueous methanol solution at room temperature to yield the crystals.

In some embodiments, crystal form A of Compound 1 hydrochloride is prepared by dissolving Compound 1 in acetone, adding concentrated hydrochloric acid, magnetically stirring, separating by suction filtration, and drying under vacuum at room temperature to yield the crystals.

Preferably, magnetic stirring is performed at room temperature for about 2 days, followed by drying under vacuum overnight.

In some embodiments, crystal form B of Compound 1 hydrochloride is prepared by dissolving Compound 1 in acetone, adding concentrated hydrochloric acid, magnetically stirring, separating by suction filtration, and drying under vacuum at room temperature to yield the crystals.

Preferably, magnetic stirring is performed at room temperature for about 4 days, followed by drying under vacuum overnight.

In some embodiments, crystal form A of Compound 1 fumarate salt is obtained by stirring fumaric acid and Compound 1 in EtOAc at room temperature to precipitate a solid and drying under vacuum at room temperature.

Preferably, stirring is performed at room temperature for about 2 days, and drying under vacuum is performed at room temperature for about 2 days.

In some embodiments, crystal form A of Compound 1 hippurate salt is prepared by adding Compound 1 to hippuric acid in acetone, magnetically stirring at room temperature, separating the solid, and drying under vacuum at room temperature to yield the crystals.

Preferably, magnetic stirring is performed for about 2 days, and drying under vacuum is performed overnight.

In some embodiments, crystal form A of Compound 1 malonate salt is prepared by stirring Compound 1 and malonic acid in acetone at room temperature, transferring the mixture to a low temperature until complete dissolution, adding an anti-solvent n-heptane to resulting in colloidization, followed by stirring in cyclic suspension at a temperature of 50~5°C to yield the crystals.

Preferably, after stirring at room temperature for 2 days, the mixture is transferred and stirred at about 5°C for about 6 days and then at -20°C for about 1 day.

In some embodiments, crystal form A of Compound 1 methanesulfonate salt is prepared by stirring Compound 1 and methanesulfonic acid in acetone at room temperature and drying under vacuum the resulting solid at room temperature to yield the crystals.

Preferably, stirring is performed at room temperature for about 2 days, and drying under vacuum is performed at room temperature for about 2 days.

In some embodiments, crystal form A of Compound 1 benzenesulfonate salt is prepared by suspension stirring a solution of Compound 1 and benzenesulfonic acid in ethanol at room temperature and drying under vacuum at room temperature to yield the crystals.

Preferably, suspension stirring is performed at room temperature for about 2 days, and drying under vacuum is performed at room temperature for about 2 days.

In some embodiments, crystal form B of Compound 1 benzenesulfonate salt is prepared by suspension stirring Compound 1 and benzenesulfonic acid in acetone at room temperature to yield the crystals.

Preferably, suspension stirring is performed at room temperature for about 2 days, and drying under vacuum is performed at room temperature for about 2 days.

In some embodiments, crystal form A of Compound 1 p-toluenesulfonate salt is prepared by suspension stirring a solution of Compound 1 and p-toluenesulfonic acid in ethanol at room temperature and drying under vacuum at room temperature to yield the crystals.

Preferably, suspension stirring is performed at room temperature for about 2 days, and drying under vacuum is performed for about 2 days.

In some embodiments, crystal form B of Compound 1 p-toluenesulfonate salt is prepared by suspension stirring Compound 1 and p-toluenesulfonic acid in ethyl acetate at room temperature and drying under vacuum at room temperature to yield the crystals.

Preferably, suspension stirring is performed at room temperature for about 2 days, and drying under vacuum is performed for about 2 days.

### 4. Use of the crystal forms of the present invention

Compound 1 described in the present invention can reduce or inhibit the activity of 3CLpro and reduce or inhibit the coronavirus replication. Therefore, the crystal forms of the present invention can be used as broad-spectrum inhibitors of coronavirus 3CLpro for the treatment of infections caused by viruses of the Coronaviridae family including SARS-CoV-2, SARS-CoV, MERS-CoV, etc.

For medical and pharmaceutical applications, the crystal forms of the present invention can be administered to patients in need in the form of any standard pharmaceutical composition. The pharmaceutical composition of the present invention comprises a pharmaceutically acceptable carrier, which can be selected from adjuvants and vehicles. The pharmaceutically acceptable carriers used in the present invention can be selected from any and all suitable solvents, diluents, vehicles, dispersing agents, suspending agents, surfactants, isotonic agents, thickening agents, emulsifiers, preservatives, solid binders and lubricants. Those skilled in the art can select a suitable carrier for the present invention from known pharmaceutically acceptable carriers according to the specific dosage form desired. For example, Remington: The Science and Practice of Pharmacy, 21st Edition, 2005, edited by D. B. Troy, Lippincott Williams & Wilkins, Philadelphia, and Encyclopedia of Pharmaceutical Technology, edited by J. Swarbrick and J. C. Boylan, 1988-1999, Marcel Dekker, New York, disclose various carriers for formulating pharmaceutical compositions and known techniques for preparing them. Non-limiting examples of suitable pharmaceutically acceptable carriers include ion exchangers, aluminum oxide, aluminum stearate, lecithin, serum proteins (such as human serum albumin), buffering substances (such as phosphates, glycine, sorbic acid and potassium sorbate), mixtures of partial glycerides of saturated vegetable fatty acids, water, salts and electrolytes (such as protamine sulfate, disodium hydrogen phosphate, potassium hydrogen phosphate, sodium chloride and zinc salts), colloidal silicon dioxide, magnesium trisilicate, polyvinylpyrrolidone, polyacrylates, waxes, polyethylene polyoxypropylene block polymers, lanolin, sugars (such as lactose, glucose and sucrose), starches (such as corn starch and potato starch), cellulose and its derivatives (such as sodium carboxymethyl cellulose, ethyl cellulose and cellulose acetate), tragacanth powder, malt, gelatin, talc, excipients (such as cocoa butter and suppository waxes), oils (such as peanut oil, cottonseed oil, safflower oil, sesame oil, olive oil, corn oil and soybean oil), glycols (such as propylene glycol and polyethylene glycol), esters (such as ethyl oleate and ethyl laurate), agar, buffering agents (such as magnesium hydroxide and aluminum hydroxide), alginic acid, pyrogen-free water, isotonic saline, Ringer's solution, ethanol, phosphate buffer solutions, non-toxic compatible lubricants (such as sodium lauryl sulfate and magnesium stearate), coloring agents, release agents, coating agents, sweetening agents, flavoring agents, perfuming agents, preservatives and antioxidants.

The pharmaceutical composition of the present invention can be administered to patients in need by any convenient route. For example, the crystal form of Compound 1, its pharmaceutically acceptable salt or the crystal form of the salt described in the present invention can be formulated into corresponding pharmaceutical preparations, so that the preparations can be administered, for example, by oral, parenteral, sublingual, topical, rectal, nasal, buccal, vaginal, transdermal, patch, pump and other routes or via an implanted reservoir.

For oral administration, any appropriate pharmaceutical carrier commonly used in the preparation of solid preparations can be used, and the crystal forms of the present invention can be formulated into solid preparations, such as syrups, suspensions, emulsions, tablets, capsules and lozenges, using formulation techniques known in the art. Examples of these carriers include magnesium stearate, starch, lactose, sucrose, cellulose, etc. Alternatively, any appropriate pharmaceutical carrier commonly used in the preparation of liquid preparations can be used, and the crystal forms of the present invention can be formulated into liquid preparations using formulation techniques known in the art. The liquid preparations of the present invention may also contain one or more suspending agents, preservatives, flavoring agents or coloring agents.

For parenteral administration, any appropriate pharmaceutical carrier commonly used in the preparation of preparations for parenteral administration can be used, and the crystal forms of the present invention can be formulated into preparations suitable for parenteral administration, such as sterile injections, freeze-dried powders, transdermal patches, aerosols, oral and nasal inhalants and suppositories, etc., using formulation techniques known in the art. Parenteral administration routes include intravenous, intraperitoneal, subcutaneous, intramuscular, transepithelial, nasal, intrapulmonary, intrathecal, rectal and topical routes. Parenteral administration can be continuous infusion over a given period.

### Examples

To better understand the content of the present invention, further illustrations are provided below in conjunction with specific examples. However, these specific examples are not intended to limit the content of the present invention.

All solvents used in the following examples are commercially available and used without further processing, unless otherwise specified.

The following abbreviations are used in the examples below:
v/v represents volume ratio;
ACN represents acetonitrile;
Acetone represents acetone;
MeOH represents methanol;
EtOAc represents ethyl acetate;
XRPD represents X-ray powder diffraction;
TGA represents thermogravimetric analysis;
DSC represents differential scanning calorimetry;
DVS represents dynamic vapor sorption;
UPLC represents ultra-performance liquid chromatography;
SGF represents simulated gastric fluid;
FaSSIF represents fasted state simulated intestinal fluid;
FeSSIF represents fed state simulated intestinal fluid.

### Example 1: Preparation of Compound 1

Amorphous form of Compound 1 in free form was prepared as a yellow solid according to the method disclosed in paragraphs [00121] to [00128] of WO2022150962A1 and used in the following examples. The specific synthesis steps are shown in Steps a to d below:

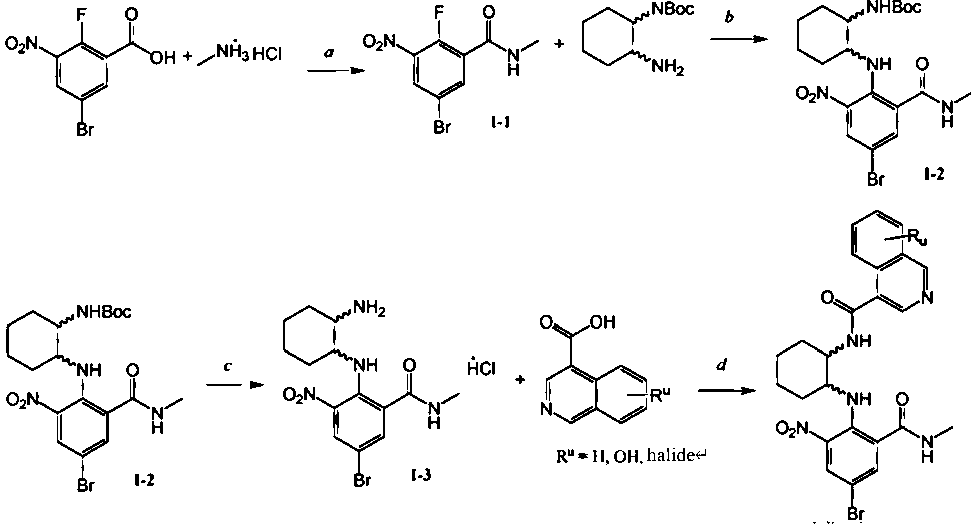

Reagents and conditions for Steps a to d as further described below are as follows: (a) 1-[bis(dimethylamino)methylene]-1H-1,2,3-triazolo[4,5-b]pyridinium 3-oxide hexafluorophosphate (HATU), N,N-diisopropylethylamine (DIPEA), CH₂Cl₂ or dichloromethane (DCM), 0°C, 2 h; (b) DIPEA, dimethylformamide (DMF), 80°C, 16 h; (c) 3M hydrogen chloride-ethyl acetate (HCl·EA), CH₂Cl₂, 1 h; (d) HATU, DIPEA, DMF, room temperature, 12 h.

### Step a: Synthesis of N-methyl-5-bromo-2-fluoro-3-nitrobenzamide (I-1)

A solution of 5-bromo-2-fluoro-3-nitrobenzoic acid (0.8 g, 3.80 mmol) in dichloromethane (20 mL) was stirred at 0°C. Then, HATU (2.0 g, 5.25 mmol), DIPEA (1.88 mL, 11.4 mmol), and methylamine hydrochloride (0.31 g, 4.5 mmol) were added to the reaction. The mixture was stirred at 0°C for 2 hours and became clear. The mixture was extracted three times with dichloromethane, and the combined organic layers were washed with a saturated brine solution. The organic phase was then dried over anhydrous Na₂SO₄ and concentrated under vacuum. Finally, the mixture was purified by chromatography to obtain Compound I-1 as a yellow solid (0.8 g, yield 76%).

### Step b: Synthesis of tert-butyl (2-((4-bromo-2-(methylcarbamoyl)-6-nitrophenyl)amino)cyclohexyl)carbamate (I-2)

A solution of Compound I-1 (0.8 g, 2.9 mmol) in DMF (15 mL) was stirred at room temperature. Then, tert-butyl ((1S,2R)-2-aminocyclohexyl)carbamate (0.75 g, 3.5 mmol) (the corresponding stereoisomer of this reagent can be used to synthesize the stereoisomer of Compound I-2) and DIPEA (1.44 mL, 8.7 mmol) were added to the reaction. The mixture was warmed to 80°C and stirred for 16 hours. The mixture was extracted three times with ethyl acetate, and the combined organic layers were washed with a saturated brine solution. The organic phase was then dried over anhydrous Na₂SO₄ and concentrated under vacuum to obtain Compound I-2 as a yellow solid, which was used without further purification.

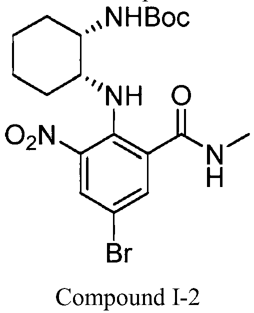

### Step c: Synthesis of 2-(2-aminocyclohexyl)amino)-5-bromo-N-methyl-3-nitrobenzamide hydrochloride (I-3)

A solution of Compound I-2 (90 mg, 0.19 mmol) (or its corresponding stereoisomer) in anhydrous dichloromethane (6 mL) was stirred at room temperature. Then, HCl (4 mL, 3 M in ethyl acetate) was added. The mixture was stirred at room temperature for 2 hours. The mixture was concentrated under vacuum to obtain Compound I-3 as a yellow solid, which was used without further purification.

### Step d: Synthesis of N-((1S,2R)-2-((4-bromo-2-(methylcarbamoyl)-6-nitrophenyl)amino)cyclohexyl)isoquinoline-4-carboxamide

A solution of the corresponding isoquinoline-4-carboxylic acid (1 equivalent) and HATU (1.5 equivalents) in anhydrous DMF (6 mL) was stirred at room temperature. Then, Compound I-3 and DIPEA (5.0 equivalents) were added. The mixture was stirred at room temperature overnight. The mixture was extracted three times with ethyl acetate, and the combined organic layers were washed with a saturated brine solution. The organic phase was then dried over anhydrous Na₂SO₄ and concentrated under vacuum. Finally, the mixture was purified by chromatography to obtain amorphous Compound 1 in free form as a yellow solid.

### Example 2: Preparation and characterization of crystal form A of Compound 1 in free form

Preparation steps of crystal form A of Compound 1 in free form are as follows:
(1) 399.9 mg of amorphous Compound 1 in free form was weighed into a 20 mL glass vial, and 10 mL of ACN/H₂O (1:1) was added.
(2) The mixture was stirred magnetically at room temperature for 2 days, and then centrifuged (10,000 rpm, 2 min) to separate the solid.
(3) The solid was dried under vacuum overnight at room temperature.

The diffraction peak data of crystal form A of Compound 1 in free form are shown in Table 1.

The XRPD pattern of crystal form A of Compound 1 in free form is shown in Figure 1A.

The TGA curve of crystal form A of Compound 1 in free form is shown in Figure 1B, with a stepwise weight loss of about 4.62% when the sample is heated to about 100°C.

The DSC curve of crystal form A of Compound 1 in free form is shown in Figure 1C, with two endothermic signals at about 107.8°C and 129.6°C (peak temperatures).

**Table 1: XRPD diffraction peak data of crystal form A of Compound 1 in free form**

| #Peak | 2θ[°] | Relative intensity [%] |
|---|---|---|
| 1 | 6.51 | 9.43 |
| 2 | 9.32 | 100.00 |
| 3 | 10.39 | 58.74 |
| 4 | 11.30 | 16.74 |
| 5 | 13.18 | 44.66 |
| 6 | 14.67 | 8.32 |
| 7 | 16.11 | 26.39 |
| 8 | 16.78 | 21.87 |
| 9 | 17.98 | 9.25 |
| 10 | 19.23 | 29.39 |
| 11 | 19.76 | 36.33 |
| 12 | 20.30 | 32.33 |
| 13 | 20.84 | 27.63 |
| 14 | 21.21 | 19.46 |
| 15 | 21.95 | 8.77 |
| 16 | 23.38 | 31.34 |
| 17 | 23.84 | 65.51 |
| 18 | 24.64 | 11.23 |
| 19 | 25.95 | 9.93 |
| 20 | 27.30 | 19.25 |
| 21 | 28.08 | 7.66 |
| 22 | 30.23 | 5.42 |
| 23 | 31.44 | 5.79 |
| 24 | 33.93 | 11.98 |
| 25 | 36.56 | 4.90 |
| 26 | 38.72 | 4.75 |

### Example 3: Preparation and characterization of crystal form B of Compound 1 in free form

Preparation steps of crystal form B of Compound 1 in free form are as follows:
(1) 399.9 mg of amorphous Compound 1 in free form was weighed into a 20 mL glass vial, and 10 mL of MeOH/H₂O (1:1) was added.
(2) The mixture was stirred magnetically at room temperature for 2 days, then centrifuged (10,000 rpm, 2 min) to separate the solid.
(3) The solid was dried under vacuum overnight at room temperature.

The diffraction peak data of crystal form B of Compound 1 in free form are shown in Table 2.

The XRPD pattern of crystal form B of Compound 1 in free form is shown in Figure 2A.

The TGA curve of crystal form B of Compound 1 in free form is shown in Figure 2B, with a weight loss of about 3.09% when the sample is heated to about 150°C.

The DSC curve of crystal form B of Compound 1 in free form is shown in Figure 2C, with 4 endothermic signals at about 50.5°C, 87.0°C, 129.5°C, and 148.4°C (peak temperatures). When crystal form B of Compound 1 in free form is heated to about 128°C and then cooled to room temperature, the XRPD results show that the crystal form remains unchanged before and after heating; the sample melts when heated to about 155°C.

**Table 2: XRPD diffraction peak data of crystal form B of Compound 1 in free form**

| #Peak | 2θ[°] | Relative intensity [%] |
|---|---|---|
| 1 | 6.76 | 63.0 |
| 2 | 8.40 | 17.7 |
| 3 | 9.07 | 100.0 |
| 4 | 10.79 | 17.4 |
| 5 | 13.50 | 52.9 |
| 6 | 13.89 | 28.8 |
| 7 | 14.61 | 6.4 |
| 8 | 15.94 | 21.5 |
| 9 | 17.10 | 3.4 |
| 10 | 19.10 | 28.1 |
| 11 | 20.16 | 29.9 |
| 12 | 20.33 | 32.5 |
| 13 | 21.03 | 30.1 |
| 14 | 21.31 | 11.6 |
| 15 | 21.68 | 28.3 |
| 16 | 22.00 | 17.9 |
| 17 | 22.36 | 25.0 |
| 18 | 22.95 | 42.4 |
| 19 | 24.10 | 20.8 |
| 20 | 24.46 | 17.8 |
| 21 | 25.13 | 14.1 |
| 22 | 26.53 | 11.7 |
| 23 | 27.14 | 24.1 |
| 24 | 27.75 | 10.1 |
| 25 | 28.46 | 18.3 |
| 26 | 29.39 | 10.5 |
| 27 | 30.78 | 8.0 |
| 28 | 31.98 | 5.2 |
| 29 | 34.20 | 11.1 |
| 30 | 35.70 | 4.8 |
| 31 | 38.80 | 3.1 |

### Example 4: Preparation and characterization of crystal form C of Compound 1 in free form

Preparation steps of crystal form C of Compound 1 in free form are as follows:
Crystal Form C of Compound 1 in free form was obtained by the slow volatilization of a solution of the amorphous sample of Compound 1 in methanol at room temperature. After standing for 4 days under ambient conditions, a mixed crystal of crystal form B of Compound 1 in free form and a small amount of crystal form C of Compound 1 in free form was obtained.

The diffraction peak data of crystal form C of Compound 1 in free form are shown in Table 3.

The XRPD pattern of crystal form C of Compound 1 in free form is shown in Figure 3.

**Table 3: XRPD diffraction peak data of crystal form C of Compound 1 in free form**

| #Peak | 2θ[°] | Relative intensity [%] |
|---|---|---|
| 1 | 8.64 | 100.00 |
| 2 | 9.23 | 13.54 |
| 3 | 12.99 | 4.53 |
| 4 | 20.29 | 3.85 |

### Example 5: Preparation and characterization of crystal form A of Compound 1 hydrochloride

Preparation steps of crystal form A of Compound 1 hydrochloride are as follows:
(1) 400.3 mg of amorphous Compound 1 in free form was weighed into a 20 mL glass vial, 10 mL of acetone was added to dissolve the compound completely, and then 65 µL of concentrated hydrochloric acid (38%) was added.
(2) The mixture was stirred magnetically at room temperature for 2 days, and the solid was separated by suction filtration.
(3) The solid was dried under vacuum overnight at room temperature, and a total of 187 mg of sample (yield: 43.7%) was collected.

The diffraction peak data of crystal form A of Compound 1 hydrochloride are shown in Table 4.

The XRPD pattern of crystal form A of Compound 1 hydrochloride is shown in Figure 4A.

The TGA curve of crystal form A of Compound 1 hydrochloride is shown in Figure 4B with a weight loss of about 1.35% when the sample is heated to about 150 °C.

The DSC curve of crystal form A of Compound 1 hydrochloride is shown in Figure 4C, with one endothermic signal at about 238.6°C (peak temperature).

**Table 4: XRPD diffraction peak data of crystal form A of Compound 1 hydrochloride**

| #Peak | 2θ[°] | Relative intensity [%] |
|---|---|---|
| | | |
| 1 | 7.84 | 18.50 |
| 2 | 11.11 | 94.45 |
| 3 | 12.74 | 6.07 |
| 4 | 13.54 | 13.14 |
| 5 | 15.07 | 4.14 |
| 6 | 15.67 | 9.58 |
| 7 | 18.22 | 3.42 |
| 8 | 18.71 | 100.00 |
| 9 | 19.70 | 10.15 |
| 10 | 20.10 | 3.30 |
| 11 | 21.24 | 37.61 |
| 12 | 21.99 | 3.41 |
| 13 | 22.29 | 17.94 |
| 14 | 22.81 | 3.81 |
| 15 | 23.72 | 40.53 |
| 16 | 25.63 | 6.04 |
| 17 | 26.03 | 10.72 |
| 18 | 26.23 | 6.43 |
| 19 | 26.91 | 3.80 |
| 20 | 27.42 | 2.30 |
| 21 | 28.22 | 4.00 |
| 22 | 29.12 | 10.40 |
| 23 | 29.94 | 3.63 |
| 24 | 30.37 | 7.50 |
| 25 | 32.50 | 3.46 |
| 26 | 33.68 | 2.55 |
| 27 | 34.70 | 2.38 |
| 28 | 37.64 | 1.67 |
| 29 | 39.12 | 1.81 |

### Example 6: Preparation and characterization of crystal form B of Compound 1 hydrochloride

Preparation steps of crystal form B of Compound 1 hydrochloride are as follows:
(1) 3.0 g of amorphous Compound 1 in free form was weighed into a 50 mL glass vial, 40 mL of acetone was added to dissolve the compound completely, and then 512 µL of concentrated hydrochloric acid was added.
(2) The mixture was stirred magnetically at room temperature for 4 days, and the solid was separated by suction filtration.
(3) The solid was dried under vacuum overnight at room temperature, and a total of 1.72 g of sample (yield: 57.3%) was collected.

The diffraction peak data of crystal form B of Compound 1 hydrochloride are shown in Table 5.

The XRPD pattern of crystal form B of Compound 1 hydrochloride is shown in Figure 5A.

The TGA curve of crystal form B of Compound 1 hydrochloride is shown in Figure 5B, with a weight loss of about 1.03% when the sample is heated to about 150 °C.

The DSC curve of crystal form B of Compound 1 hydrochloride is shown in Figure 5C, with one endothermic signal at about 248.7 °C (peak temperature).

**Table 5: XRPD diffraction peak data of crystal form B of Compound 1 hydrochloride**

| #Peak | 2θ[°] | Relative intensity [%] |
|---|---|---|
| 1 | 12.22 | 31.72 |
| 2 | 13.72 | 11.77 |
| 3 | 14.10 | 7.20 |
| 4 | 15.04 | 6.46 |
| 5 | 17.15 | 6.48 |
| 6 | 18.37 | 100.00 |
| 7 | 18.79 | 16.89 |
| 8 | 19.26 | 6.89 |
| 9 | 20.28 | 11.84 |
| 10 | 20.93 | 3.05 |
| 11 | 21.39 | 21.88 |
| 12 | 21.98 | 4.85 |
| 13 | 22.99 | 22.87 |
| 14 | 24.11 | 38.17 |
| 15 | 24.64 | 9.47 |
| 16 | 25.03 | 17.19 |
| 17 | 25.59 | 39.82 |
| 18 | 26.03 | 7.50 |
| 19 | 26.45 | 6.35 |
| 20 | 26.92 | 11.15 |
| 21 | 28.27 | 12.64 |
| 22 | 29.83 | 9.73 |
| 23 | 30.77 | 5.10 |
| 24 | 31.96 | 3.01 |
| 25 | 33.05 | 4.20 |
| 26 | 35.43 | 4.91 |
| 27 | 37.20 | 5.08 |

### Example 7: Preparation and characterization of crystal form A of Compound 1 fumarate salt

Preparation steps of crystal form A of Compound 1 fumarate salt are as follows:
(1) approximately 160 mg of amorphous Compound 1 in free form and 18.5 mg of fumaric acid were weighed into a 3 mL glass vial, and 2 mL of EtOAc was added.
(2) The mixture was stirred magnetically at room temperature for 3 hours, then seed crystals was added.
(3) The mixture was stirred magnetically at room temperature for further 4 hours, then was centrifuged (10,000 rpm, 2 min) to separate the solid.
(4) The solid was dried under vacuum overnight at room temperature, and a total of 140 mg of sample (yield: 78.4%) was collected.

Alternatively,
(1) 399.5 mg of amorphous Compound 1 in free form and 88.0 mg of fumaric acid were weighed into a 20 mL glass vial, and 10 mL of EtOAc was added.
(2) The mixture was stirred magnetically at room temperature for 2 days, and then the solid was separated by suction filtration.
(3) The solid was dried under vacuum overnight at room temperature, and a total of 292 mg of sample (yield: 59.9%) was collected.

The diffraction peak data of crystal form A of Compound 1 fumarate salt are shown in Table 6.

The XRPD pattern of crystal form A of Compound 1 fumarate salt is shown in Figure 6A.

The TGA/DSC curve of crystal form A of Compound 1 fumarate salt is shown in Figure 6B, with a weight loss of about 1.10% when the sample is heated to about 160°C, and one sharp endothermic peak at about 187°C (peak temperature).

**Table 6: XRPD diffraction peak data of crystal form A of the fumarate salt (826709-47-B3)**

| #Peak | 2θ[°] | Relative intensity [%] |
|---|---|---|
| 1 | 5.91 | 16.58 |
| 2 | 9.19 | 100.00 |
| 3 | 10.00 | 9.41 |
| 4 | 10.92 | 62.08 |
| 5 | 11.97 | 55.14 |
| 6 | 12.76 | 22.59 |
| 7 | 14.60 | 24.24 |
| 8 | 15.94 | 23.04 |
| 9 | 17.35 | 13.30 |
| 10 | 18.66 | 63.47 |
| 11 | 18.90 | 40.18 |
| 12 | 19.48 | 82.19 |
| 13 | 20.01 | 66.73 |
| 14 | 20.21 | 41.47 |
| 15 | 20.49 | 12.93 |
| 16 | 21.21 | 9.68 |
| 17 | 21.94 | 7.34 |
| 18 | 23.28 | 59.09 |
| 19 | 23.49 | 46.36 |
| 20 | 23.68 | 44.79 |
| 21 | 24.34 | 15.48 |
| 22 | 24.95 | 10.99 |
| 23 | 25.70 | 12.28 |
| 24 | 26.21 | 14.22 |
| 25 | 27.30 | 5.28 |
| 26 | 27.98 | 22.11 |
| 27 | 28.43 | 19.39 |
| 28 | 29.21 | 8.70 |
| 29 | 29.72 | 8.81 |
| 30 | 30.41 | 5.03 |
| 31 | 31.21 | 13.56 |
| 32 | 32.19 | 6.32 |
| 33 | 33.44 | 5.87 |
| 34 | 34.94 | 9.21 |
| 35 | 36.77 | 7.16 |

### Example 8: Preparation and characterization of crystal form A of Compound 1 hippurate salt

Preparation steps of crystal form A of Compound 1 hippurate salt are as follows:
(1) 400.0 mg of amorphous Compound 1 in free form and 136.2 mg of hippuric acid were weighed into a 20 mL glass vial, and 10 mL of acetone was added.
(2) The mixture was stirred magnetically at room temperature for 2 days, then the solid was separated by suction filtration.
(3) The solid was dried under vacuum overnight at room temperature, and a total of 303 mg of sample (yield: 61.5%) was collected.

The diffraction peak data of crystal form A of Compound 1 hippurate salt are shown in Table 7.

The XRPD pattern of crystal form A of Compound 1 hippurate salt is shown in Figure 7A.

The TGA curve of crystal form A of Compound 1 hippurate salt is shown in Figure 7B, with a weight loss of about 0.72% when the sample is heated to about 150°C.

The DSC curve of crystal form A of Compound 1 hippurate salt is shown in Figure 7C, with one sharp endothermic peak at about 151.8°C (peak temperature).

**Table 7: XRPD diffraction peak data of crystal form A of Compound 1 hippurate salt**

| #Peak | 2θ[°] | Relative intensity [%] |
|---|---|---|
| 1 | 4.90 | 100.00 |
| 2 | 9.09 | 29.29 |
| 3 | 10.73 | 71.26 |
| 4 | 11.74 | 5.41 |
| 5 | 13.06 | 3.40 |
| 6 | 13.59 | 7.79 |
| 7 | 13.87 | 16.19 |
| 8 | 14.64 | 27.23 |
| 9 | 14.99 | 3.63 |
| 10 | 17.10 | 4.73 |
| 11 | 17.34 | 5.82 |
| 12 | 18.02 | 8.71 |
| 13 | 18.41 | 19.86 |
| 14 | 18.74 | 4.79 |
| 15 | 19.56 | 28.89 |
| 16 | 19.82 | 26.18 |
| 17 | 20.08 | 6.48 |
| 18 | 20.28 | 4.71 |
| 19 | 21.01 | 28.82 |
| 20 | 21.21 | 87.39 |
| 21 | 21.65 | 9.84 |
| 22 | 22.22 | 19.42 |
| 23 | 22.74 | 21.81 |
| 24 | 23.09 | 7.25 |
| 25 | 23.96 | 21.46 |
| 26 | 24.50 | 15.87 |
| 27 | 24.84 | 21.94 |
| 28 | 25.22 | 8.11 |
| 29 | 25.44 | 12.62 |
| 30 | 25.67 | 8.86 |
| 31 | 26.17 | 2.32 |
| 32 | 26.57 | 2.61 |
| 33 | 27.25 | 4.71 |
| 34 | 28.03 | 15.28 |
| 35 | 28.81 | 7.87 |
| 36 | 29.09 | 7.73 |
| 37 | 29.47 | 9.65 |
| 38 | 31.46 | 3.95 |
| 39 | 32.46 | 4.57 |
| 40 | 34.09 | 3.81 |
| 41 | 35.06 | 6.96 |
| 42 | 35.77 | 4.96 |
| 43 | 36.18 | 3.53 |
| 44 | 37.25 | 2.86 |
| 45 | 38.90 | 5.87 |

### Example 9: Preparation and characterization of crystal form A of Compound 1 malonate salt

Preparation steps of crystal form A of Compound 1 malonate salt are as follows:
(1) About 400 mg of amorphous Compound 1 in free form and an equimolar amount of malonic acid in acetone were stirred at room temperature for 2 days.
(2) The mixture was transferred to 5°C and stirred for 6 days, then transferred to -20°C and stirred for 1 day, and the mixture remained clear.
(3) An anti-solvent n-heptane was added, and a colloidal substance was formed.
(4) The mixture was transferred to a temperature of 50~5°C and stirred in cyclic suspension to precipitate solids.

The diffraction peak data of crystal form A of Compound 1 malonate salt are shown in Table 8.

The XRPD pattern of crystal form A of Compound 1 malonate salt is shown in Figure 8A.

The TGA curve of crystal form A of Compound 1 malonate salt is shown in Figure 8B, with a weight loss of about 3.26% when the sample is heated to about 100°C, and about 10.83% when further heated to about 180°C.

The DSC curve of crystal form A of Compound 1 malonate salt is shown in Figure 8C, with three endothermic signals at about 53.0°C, 110.6°C, and 150.9°C (peak temperatures).

**Table 8: XRPD diffraction peak data of crystal form A of Compound 1 malonate salt**

| #Peak | 2θ[°] | Relative intensity [%] |
|---|---|---|
| 1 | 5.36 | 51.68 |
| 2 | 8.37 | 100.00 |
| 3 | 9.90 | 23.13 |
| 4 | 11.54 | 23.56 |
| 5 | 16.99 | 21.12 |
| 6 | 20.49 | 38.42 |
| 7 | 21.39 | 51.73 |
| 8 | 22.50 | 13.60 |
| 9 | 23.75 | 46.75 |
| 10 | 25.09 | 19.58 |
| 11 | 27.35 | 10.66 |

### Example 10: Preparation and characterization of crystal form A of Compound 1 methanesulfonate salt

Preparation steps of crystal form A of Compound 1 methanesulfonate salt are as follows:
(1) about 400 mg of amorphous Compound 1 in free form and an equimolar amount of methanesulfonic acid in acetone were stirred at room temperature for 2 days.
(2) The resulting solid was dried under vacuum at room temperature for 2 days to obtain the methanesulfonate salt crystal form A.

The diffraction peak data of crystal form A of Compound 1 methanesulfonate salt are shown in Table 9.

The XRPD pattern of crystal form A of Compound 1 methanesulfonate salt is shown in Figure 9A.

The TGA curve of crystal form A of Compound 1 methanesulfonate salt is shown in Figure 9B, with a stepwise weight loss of about 4.43% when the sample is heated to about 160°C.

The DSC curve of crystal form A of Compound 1 methanesulfonate salt is shown in Figure 9C, with two endothermic signals at about 96.7°C and 145.5°C (peak temperatures).

**Table 9: XRPD diffraction peak data of crystal form A of Compound 1 methanesulfonate salt**

| #Peak | 2θ[°] | Relative intensity [%] |
|---|---|---|
| 1 | 8.40 | 100.00 |
| 2 | 11.59 | 5.65 |
| 3 | 16.95 | 14.41 |
| 4 | 21.30 | 23.54 |
| 5 | 25.31 | 9.32 |

### Example 11: Preparation and characterization of crystal form A of Compound 1 benzenesulfonate salt

Preparation Steps of crystal form A of Compound 1 benzenesulfonate salt are as follows:
(1) About 400 mg of amorphous Compound 1 in free form and an equimolar amount of benzenesulfonic acid in EtOH/H₂O (19:1, v/v) were stirred in suspension at room temperature for 2 days.
(2) The solid was dried under vacuum at room temperature for 2 days to obtain the benzenesulfonate salt crystal form A.

The diffraction peak data of crystal form A of Compound 1 benzenesulfonate salt are shown in Table 10.

The XRPD pattern of crystal form A of Compound 1 benzenesulfonate salt is shown in Figure 10A.

The TGA curve of crystal form A of Compound 1 benzenesulfonate salt is shown in Figure 10B, with a weight loss of about 3.34% when the sample is heated to about 120°C, and about 5.97% when further heated to about 180°C.

The DSC curve of crystal form A of Compound 1 benzenesulfonate salt is shown in Figure 10C, with two endothermic signals at about 65.3°C and 153.7°C (peak temperatures).

**Table 10: XRPD diffraction peak data of crystal form A of Compound 1 benzenesulfonate salt**

| #Peak | 2θ[°] | Relative intensity [%] |
|---|---|---|
| 1 | 8.62 | 96.6 |
| 2 | 11.40 | 22.9 |
| 3 | 11.95 | 13.0 |
| 4 | 13.63 | 11.3 |
| 5 | 14.71 | 9.1 |
| 6 | 16.06 | 17.2 |
| 7 | 16.67 | 15.6 |
| 8 | 17.24 | 24.9 |
| 9 | 18.82 | 9.3 |
| 10 | 19.42 | 42.1 |
| 11 | 20.69 | 25.3 |
| 12 | 21.60 | 14.9 |
| 13 | 22.28 | 23.0 |
| 14 | 22.88 | 14.8 |
| 15 | 23.62 | 25.3 |
| 16 | 24.28 | 43.9 |
| 17 | 24.66 | 16.2 |
| 18 | 25.62 | 100.0 |
| 19 | 26.00 | 42.5 |
| 20 | 27.07 | 25.1 |
| 21 | 28.81 | 8.8 |
| 22 | 30.41 | 9.4 |
| 23 | 32.30 | 6.1 |
| 24 | 33.45 | 3.1 |

### Example 12: Preparation and characterization of crystal form B of Compound 1 benzenesulfonate salt

Preparation steps of crystal form B of Compound 1 benzenesulfonate salt are as follows:
(1) About 400 mg of amorphous Compound 1 in free form and an equimolar amount of benzenesulfonic acid in acetone were stirred in suspension at room temperature for 2 days.
(2) The solid was dried under vacuum at room temperature for 2 days to obtain the benzenesulfonate salt crystal form B.

The diffraction peak data of crystal form B of Compound 1 benzenesulfonate salt are shown in Table 11.

The XRPD pattern of crystal form B of Compound 1 benzenesulfonate salt is shown in Figure 11A.

The TGA curve of crystal form B of Compound 1 benzenesulfonate salt is shown in Figure 11B, with a weight loss of about 3.67% when the sample is heated to about 180°C.

The DSC curve of crystal form B of Compound 1 benzenesulfonate salt is shown in Figure 11C, with one sharp endothermic peak at about 194.1°C (onset temperature).

**Table 11: XRPD diffraction peak data of crystal form B of Compound 1 benzenesulfonate salt**

| #Peak | 2θ[°] | Relative intensity [%] |
|---|---|---|
| 1 | 8.89 | 100.00 |
| 2 | 9.93 | 10.81 |
| 3 | 13.36 | 7.29 |
| 4 | 15.35 | 7.03 |
| 5 | 17.05 | 7.40 |
| 6 | 17.75 | 9.40 |
| 7 | 18.49 | 6.16 |
| 8 | 20.49 | 9.13 |
| 9 | 22.35 | 15.43 |
| 10 | 25.58 | 5.77 |
| 11 | 26.99 | 5.18 |
| 12 | 28.69 | 5.21 |

### Example 13: Preparation and characterization of crystal form A of Compound 1 p-toluenesulfonate salt

Preparation of crystal form A of Compound 1 p-toluenesulfonate salt are as follows:
(1) About 400 mg of amorphous Compound 1 in free form and an equimolar amount of p-toluenesulfonic acid in EtOH/H₂O (19:1, v/v) were stirred in suspension at room temperature for 2 days.
(2) The solid was dried under vacuum at room temperature for 2 days.

The diffraction peak data of crystal form A of Compound 1 p-toluenesulfonate salt are shown in Table 12.

The XRPD pattern of crystal form A of Compound 1 p-toluenesulfonate salt is shown in Figure 12A.

The TGA curve of crystal form A of Compound 1 p-toluenesulfonate salt is shown in Figure 12B, with a weight loss of about 3.64% when the sample is heated to about 130°C, and about 3.17% when further heated to about 170°C.

The DSC curve of crystal form A of Compound 1 p-toluenesulfonate salt is shown in Figure 12C, with two endothermic signals at about 122.7°C and 150.5°C (peak temperatures).

**Table 12: XRPD diffraction peak data of crystal form A of Compound 1 p-toluenesulfonate salt**

| #Peak | 2θ[°] | Relative intensity [%] |
|---|---|---|
| 1 | 8.38 | 100.00 |
| 2 | 11.42 | 19.51 |
| 3 | 11.93 | 10.82 |
| 4 | 14.59 | 5.92 |
| 5 | 16.09 | 13.92 |
| 6 | 16.45 | 10.93 |
| 7 | 16.78 | 24.06 |
| 8 | 18.64 | 10.38 |
| 9 | 19.21 | 33.21 |
| 10 | 20.55 | 19.53 |
| 11 | 21.73 | 16.82 |
| 12 | 22.29 | 17.20 |
| 13 | 24.16 | 38.33 |
| 14 | 25.75 | 82.99 |
| 15 | 27.09 | 25.96 |
| 16 | 30.07 | 7.51 |

### Example 14: Preparation and characterization of crystal form B of Compound 1 p-toluenesulfonate salt

Preparation steps of crystal form B of Compound 1 p-toluenesulfonate salt are as follows:
(1) About 400 mg of amorphous Compound 1 in free form and an equimolar amount of p-toluenesulfonic acid in EtOAc were stirred in suspension at room temperature for 2 days.
(2) The resulting solid was dried under vacuum at room temperature for 2 days.

The diffraction peak data of crystal form B of Compound 1 p-toluenesulfonate salt are shown in Table 13.

The XRPD pattern of crystal form B of Compound 1 p-toluenesulfonate salt is shown in Figure 13A.

The TGA curve of crystal form B of Compound 1 p-toluenesulfonate salt is shown in Figure 13B, with a weight loss of about 5.05% when the sample is heated to about 140°C.

The DSC curve of crystal form B of Compound 1 p-toluenesulfonate salt is shown in Figure 13C, with one endothermic signal at about 154.6°C (peak temperature).

**Table 13: XRPD diffraction peak data of crystal form B of Compound 1 p-toluenesulfonate salt**

| #Peak | 2θ[°] | Relative intensity [%] |
|---|---|---|
| 1 | 5.61 | 20.83 |
| 2 | 7.76 | 32.22 |
| 3 | 9.22 | 100 |
| 4 | 10.88 | 7.07 |
| 5 | 11.99 | 26.96 |
| 6 | 14.22 | 19.98 |
| 7 | 15.91 | 6.93 |
| 8 | 17.69 | 6.82 |
| 9 | 18.44 | 26.25 |
| 10 | 21.26 | 33.44 |
| 11 | 23.33 | 16.05 |

### Example 15: Dynamic solubility test

The solubilities of the crystal forms obtained from Examples 2-14 in water (H₂O) and biological vehicles (simulated gastric fluid (SGF), fasted state simulated intestinal fluid (FaSSIF), and fed state simulated intestinal fluid (FeSSIF)) were tested at 1/2/4/24 hours. The experimental results are shown in Table 14.

Specific procedures of the test are as follows:
1) About 20-40 mg of the crystal forms from Examples 2-14 were weighed into 5 mL glass vials, and 4 mL of the corresponding medium (H₂O, SGF, FaSSIF, and FeSSIF) was added to the vials, respectively.
2) The vials were mixed by rotation on a rotary incubator at 37°C at a speed of 25 rpm. Sampling time points are 1, 2, 4, and 24 hours.
3) At each sampling time point, about 0.8 mL of the suspension was taken into a centrifuge tube, and separated by centrifugation (12,000 rpm, 5 min, 37°C).
4) The supernatant was filtered through a PTFE membrane (pore size: 0.45 µm), the filtrate was used for testing solubility and pH, and the solid was subjected to XRPD analysis.

### Experimental results:

**Table 14: Results of the dynamic solubility evaluation**

| **Sample** | **Medium** | **1 hr** | | | **2 hrs** | | | **4 hrs** | | | **24 hrs** | | |
|---|---|---|---|---|---|---|---|---|---|---|---|---|---|
| | | **S** | **pH** | **FC** | **S** | **pH** | **FC** | **S** | **pH** | **FC** | **S** | **pH** | **FC** |
| Free form amorphous | H₂O | * | 8.5 | No | * | 8.7 | No | * | 8.7 | No | * | 8.7 | No |
| | SGF | *** | 1.9 | No | *** | 1.9 | No | *** | 1.9 | No | *** | 1.8 | No |
| | FaSSIF | * | 6.5 | No | * | 6.5 | No | * | 6.6 | No | * | 6.6 | No |
| | FeSSIF | ** | 5.1 | No | ** | 5.1 | Yes | ** | 5.1 | Yes | ** | 5.1 | Yes |
| Free form crystal form A | H₂O | * | 8.4 | No | * | 8.5 | No | * | 8.7 | No | * | 8.7 | No |
| | SGF | ** | 1.7 | No | ** | 1.7 | No | ** | 1.8 | No | ** | 1.7 | No |
| | FaSSIF | * | 6.5 | No | * | 6.5 | No | * | 6.5 | No | * | 6.5 | No |
| | FeSSIF | ** | 5.1 | No | ** | 5.1 | No | ** | 5.2 | No | ** | 5.1 | No |
| Free form crystal form B | H₂O | * | 8.6 | No | * | 8.6 | No | * | 8.7 | No | * | 8.7 | No |
| | SGF | *** | 1.9 | No | *** | 1.9 | No | *** | 1.9 | No | *** | 1.9 | No |
| | FaSSIF | * | 6.6 | No | * | 6.5 | No | * | 6.6 | No | * | 6.5 | No |
| | FeSSIF | ** | 5.1 | No | ** | 5.1 | No | ** | 5.1 | No | ** | 5.1 | No |
| Hydrochloride salt crystal form A | H₂O | ** | 7.9 | No | ** | 6.6 | No | ** | 6 | No | *** | 1.8 | No |
| | SGF | *** | 1.7 | No | *** | 1.6 | No | *** | 1.6 | No | *** | 1.3 | No |
| | FaSSIF | * | 6.5 | No | * | 6.5 | No | * | 6.6 | No | * | 6.3 | No |
| | FeSSIF | ** | 5.1 | No | ** | 5 | No | ** | 5 | No | ** | 5 | No |
| Hydrochloride salt crystal form B | H₂O | * | 7.4 | No | ** | 6.8 | No | ** | 4.4 | No | *** | 1.8 | No |
| | SGF | *** | 1.7 | No | *** | 1.6 | No | *** | 1.5 | No | *** | 1.4 | No |
| | FaSSIF | * | 6.5 | No | * | 6.5 | No | * | 6.5 | No | * | 6.4 | No |
| | FeSSIF | ** | 5 | No | ** | 5 | No | ** | 5 | No | ** | 4 | No |
| Fumarate salt crystal form A | H₂O | ** | 2.6 | No | ** | 2.6 | No | ** | 2.6 | No | ** | 2.5 | No |
| | SGF | ** | 1.7 | No | ** | 1.7 | No | ** | 1.6 | No | ** | 1.6 | No |
| | FaSSIF | ** | 3.6 | No | ** | 3.5 | No | ** | 3.5 | No | ** | 3.4 | No |
| | FeSSIF | ** | 4.9 | No | ** | 4.7 | No | ** | 4.7 | No | ** | 4.7 | No |
| Hippurate salt crystal form A | H₂O | ** | 3.5 | No | ** | 3.6 | No | ** | 3.4 | No | ** | 3.3 | No |
| | SGF | *** | 1.8 | No | *** | 1.8 | No | *** | 1.7 | No | *** | 1.7 | No |
| | FaSSIF | * | 6 | No | * | 6 | No | * | 5.9 | No | * | 5.6 | No |
| | FeSSIF | * | 5.1 | No | ** | 5.1 | No | ** | 5.1 | No | ** | 5.1 | No |

| | | | | | | | | | | | | | |
|---|---|---|---|---|---|---|---|---|---|---|---|---|---|
| S: solubility (mg/mL), in free form. FC: Form change. *: S<0.1mg/mL: **: 0.1mg/mL≤S<1mg/mL; ***: 1mg/mL≤S. | | | | | | | | | | | | | |

The experimental results show that:
1) In water (H₂O), the solubility of the 4 salt forms is slightly higher than that of the 3 free form samples.
2) In SGF, the solubility of the hydrochloride salt crystal forms A and B is significantly higher than that of the fumarate salt, the hippurate salt, and the 3 free form samples.
3) In FaSSIF, the solubility of the fumarate salt crystal form A is slightly higher than that of the hydrochloride salt crystal forms A/B, the hippurate salt crystal form A, the free form crystal forms A/B, and the free form amorphous.
4) In FeSSIF, there is no significant difference in solubility among the free form amorphous, the free form crystal form B, the hydrochloride salt crystal forms A/B, the fumarate salt crystal form A, and the hippurate salt crystal form A.
5) The XRPD results show that the free amorphous form undergoes a form change after being suspended in FeSSIF for 2 hours. The free crystal forms A/B and the 4 salt forms do not undergo any form change in all media.

### Example 16: Hygroscopicity test

In accordance with the Guidelines for Hygroscopicity Tests of Drugs in the Chinese Pharmacopoeia (2020 Edition), a DVS test was conducted on the crystal forms from Examples 2-14 to determine their hygroscopicity. The hygroscopicity test results are shown in Table 15.

### Experimental results:

**Table 15: Results of the hygroscopicity test**

| Crystal form | Water absorption weight gain (wt%) at 25 °C/80% RH | Form change after the DVS |
|---|---|---|
| Free form amorphous form | >1.5 | No |
| Free form crystal form A | <1 | No |
| Free form crystal form B | <0.5 | No |
| Hydrochloride crystal form A | <0.5 | No |
| Hydrochloride crystal form B | <0.5 | No |
| Fumarate salt crystal form A | <0.1 | No |
| Hippurate salt crystal form A | <0.5 | No |

The DVS results show that all the crystal forms tested exhibit almost no hygroscopicity, representing a significant advantage over the free form amorphous form. Moreover, no from change is observed for all the crystal forms after the DVS test.

### Example 17: Solid-state stability test

An appropriate amount of the crystal form samples from Examples 2-14 were weighed respectively, and placed open under the conditions of 25°C/60%RH and 40°C/75%RH for 7-10 days for stability test. For the solid samples separated under different conditions, XRPD was used to test the crystal form for evaluation of the physical stability, and UPLC was used to test the purity for evaluation of the chemical stability. The solid-state stability test results are shown in Table 16.
The experimental results are as shown in the following table:

**Table 16: Solid-state stability results of free amorphous form, free crystal forms and preferential salt forms**

| Salt form (Sample No.) | Initial purity (area%) | 25 °C/60% RH/Open | | 40 °C/75%RH/Open | |
|---|---|---|---|---|---|
| | | Purity (area%) | Form change | Purity (area%) | Form change |
| Free form Amorphous form | 97.93 | 98.04 | No | 97.03 | No |
| Free form Crystal form A | 98.03 | 98.50* | No | 98.59* | No |
| Free form Crystal form B | 97.96 | 98.17* | No | 98.24* | No |
| Hydrochloride Crystal form A | 99.72 | 99.74 | No | 99.74 | No |
| Hydrochloride crystal form B | 99.43 | 99.43 | No | 99.37 | No |
| Fumarate salt Crystal form A | 98.73 | 98.74 | No | 98.76 | No |
| Hippurate salt Crystal form A | 98.64 | 98.67 | No | 98.66 | No |

| | | | | | |
|---|---|---|---|---|---|
| *: The area percentage of the impurity with RRT = 1.11 decreased. ^{#}: The hydrochloride salt crystal form B and the fumarate salt crystal form A were placed open under the two conditions for 7 days, while the remaining samples were placed open under the two conditions for 10 days. | | | | | |

The solid-state stability results show that all the crystal form samples tested exhibit no form change or purity reduction after being placed open at 25°C/60% RH and 40°C/75% RH for 7-10 days, indicating that they all have good physical and chemical stability under the evaluation conditions.

### Instruments and methods

### 1. X-ray powder diffraction (also referred to as powder X-ray diffractometer, XRPD) method

XRPD results are collected using X'Pert3 and Empyrean X-ray powder diffractometers, with the scanning parameters as shown in Table 17.

**Table 17: XRPD test parameters**

| Mode | Conventional reflection | Variable-temperature XRPD |
|---|---|---|
| X-ray | Cu, kα; Kα1 (Ⓐ): 1.540598, Kα2 (Ⓐ): 1.544426 | |
| | Intensity ratio Kα2/Kα1: 0.50 | |
| X-ray tube settings | 45 kV, 40 mA | 45 kV, 40 mA |
| Divergent slit | 1/8° | 1/8° |
| Scanning mode | Continuous | Continuous |
| Scanning range (°2TH) | 3° ~ 40° | 3° ~ 40° |
| Scanning step length (°2TH) | 0.0263 | 0.0167 |
| Scanning time of each step(s) | 46.67 | 33.02 |
| Scanning time(s) | 5 min 04 s | 10 min 12 s |

### 2. Thermogravimetric analysis (TGA) and differential scanning calorimetry (DSC)

TGA results are collected using a TA Discovery 5500 thermogravimetric analyzer, and DSC results are collected using a TA Discovery 2500 differential scanning calorimeter, with the test parameters as shown in Table 18.

**Table 18: TGA and DSC test parameters**

| Parameter | TGA | DSC |
|---|---|---|
| Method | Linear temperature rise | Linear temperature rise |
| Sample plate | Aluminum plate, open | Aluminum plate, cover |
| Temperature range | Room temperature-300°C | 25-250°C |
| Heating rate | 10°C/min | 10°C/min |
| Protective gas | Nitrogen | Nitrogen |

### 3. Dynamic vapor sorption (DVS)

Dynamic vapor sorption (DVS) curve was collected using a DVS Intrinsic instrument from SMS (Surface Measurement Systems). At 25°C, the relative humidity was calibrated using the deliquescence points of LiCl, Mg(NO₃)₂ and KCl. The DVS test parameters are shown in Table 19.

**Table 19: DVS test parameters**

| Parameter | DVS |
|---|---|
| Temperature | 25°C |
| Sample amount | 10-30 mg |
| Protective gas and flow rate | N₂, 200 mL/min |
| dm/dt | 0.002%/min |
| Minimum dm/dt equilibration time | 10 min |
| Maximum equilibrium time | 180 min |
| RH range | Room humidity~95%RH~0%RH~95%RH (Crystal Form A of Compound 1) |
| | 0%RH~95%RH~0%RH (other samples) |
| RH gradient | 10%RH (0%RH~90%RH & 90%RH~0%RH) |
| | 5%RH (90%RH~95%RH & 95%RH~90%RH) |

### 4. Ultra-performance liquid chromatography (UPLC)

In the experiment, purity, solubility and molar ratio were tested using a Waters H-Class UPLC ultra-performance liquid chromatograph, with the analysis conditions shown in Table 20 and Table 21.

**Table 20: UPLC operation conditions for testing purity**

| UPLC | Waters H-Class UPLC | |
|---|---|---|
| Chromatographic column | ZORBAX Eclipse XDB-C18 RRHT, 50×4.6 mm, 1.8 µm | |
| | A: 0.04% TFA in H₂O | |
| Mobile phase | B: 0.02% TFA in ACN | |
| | Time (min) | %B |
| | 0.0 | 10 |
| | 15.0 | 80 |
| | 16.0 | 80 |
| Elution gradient | 16.1 | 10 |
| | 18.0 | 10 |
| Run time | 18.0 min | |
| Post runtime | 0.0 min | |
| Mobile phase flow rare | 1.0 mL/min | |
| Sample injection volume | 3 µL | |
| Detection wavelength | 220 nm | |
| Column temperature | 30°C | |
| Sample injector temperature | Room temperature | |
| Diluent | ACN/H₂O (1:1, v/v) | |

**Table 21: UPLC operation conditions for testing solubility and molar ratio**

| UPLC | Waters H-Class UPLC | |
|---|---|---|
| Chromatographic column | ZORBAX Eclipse XDB-C18 RRHT, 50×4.6 mm, 1.8 µm | |
| Mobile phase | A: 0.04% TFA in H₂O | |
| | B: 0.02% TFA in ACN | |
| | Time (min) | %B |
| | 0.0 | 10 |
| | 5.0 | 80 |
| Elution gradient | 6.0 | 80 |
| | 6.1 | 10 |
| | 8.0 | 10 |
| Run time | 8.0 min | |
| Post runtime | 0.0 min | |
| Mobile phase flow rare | 1.0 mL/min | |
| Sample injection volume | 0.8 µL | |
| Detection wavelength | 220 nm | |
| Column temperature | 30°C | |
| Sample injector temperature | Room temperature | |
| Diluent | ACN/H₂O (1:1, v/v) | |

## Claims

1. A crystal form of a compound of the following formula or a pharmaceutically acceptable salt thereof

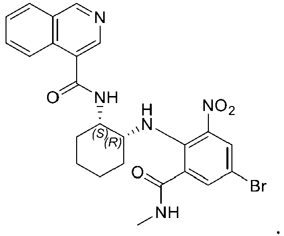

2. The crystal form as claimed in claim 1, wherein the salt is a salt formed by the compound with an acid selected from hydrochloric acid, fumaric acid, hippuric acid, malonic acid, methanesulfonic acid, benzenesulfonic acid or p-toluenesulfonic acid.

3. The crystal form as claimed in claim 2, which is **characterized in that** the salt is formed by the compound and the acid in a molar ratio of 1:0.5-2.

4. The crystal form as claimed in claim 1, which is **characterized in that** the crystal form exhibits an XRPD pattern having diffraction peaks at the 2θ angles of approximately 9.32, 10.39, 13.18, 19.76, and 23.84.

5. The crystal form as claimed in claim 1, which is **characterized in that** the crystal form exhibits an XRPD pattern having diffraction peaks at the 20 angles of approximately 6.76, 9.07, 13.50, 20.33, and 22.95.

6. The crystal form as claimed in claim 1, which is **characterized in that** the crystal form exhibits an XRPD pattern having diffraction peaks at the 2θ angles of approximately 8.64, 9.23, and 12.99.

7. The crystal form as claimed in claim 1, which is **characterized in that** the crystal form exhibits an XRPD pattern having diffraction peaks at the 20 angles of approximately 7.84, 11.11, 18.71, 21.24, and 23.72.

8. The crystal form as claimed in claim 1, which is **characterized in that** the crystal form exhibits an XRPD pattern having diffraction peaks at the 2θ angles of approximately 12.22, 18.37,

9. The crystal form as claimed in claim 1, which is **characterized in that** the crystal form exhibits an XRPD pattern having diffraction peaks at the 20 angles of approximately 9.19, 10.92, 18.66, 19.48, and 20.01.

10. The crystal form as claimed in claim 1, which is **characterized in that** the crystal form exhibits an XRPD pattern having diffraction peaks at the 20 angles of approximately 4.90, 9.09, 10.73, 19.56, and 21.21.

11. The crystal form as claimed in claim 1, which is **characterized in that** the crystal form exhibits an XRPD pattern having diffraction peaks at the 20 angles of approximately 5.36, 8.37, and 21.39.

12. The crystal form as claimed in claim 1, which is **characterized in that** the crystal form exhibits an XRPD pattern having diffraction peaks at the 20 angles of approximately 8.40, 16.95, and 21.30.

13. The crystal form as claimed in claim 1, which is **characterized in that** the crystal form exhibits an XRPD pattern having diffraction peaks at the 20 angles of approximately 8.62, 19.42, 24.28, 25.62, and 26.00.

14. The crystal form as claimed in claim 1, which is **characterized in that** the crystal form exhibits an XRPD pattern having diffraction peaks at the 2θ angles of approximately 8.89, 9.93, and 22.35.

15. The crystal form as claimed in claim 1, which is **characterized in that** the crystal form exhibits an XRPD pattern having diffraction peaks at the 20 angles of approximately 8.38, 19.21, 24.16, 25.75, and 27.09.

16. The crystal form as claimed in claim 1, which is **characterized in that** the crystal form exhibits an XRPD pattern having diffraction peaks at the 20 angles of approximately 7.76, 9.22, 11.99, 18.44, and 21.26.

17. A composition, comprising the crystal form as claimed in any of claims 1-16, wherein the crystal form accounts for more than 50%, preferably more than 75%, more preferably more than

18. A pharmaceutical composition, comprising the crystal form as claimed in any of claims 1-16 and at least one pharmaceutically acceptable carrier.

19. Use of the crystal form as claimed in any of claims 1-16 in the preparation of a medicament for treating diseases caused by coronaviruses or symptoms thereof; preferably, the disease is selected from COVID-19, SARS and MERS.

20. The crystal form as claimed in any of claims 1-16 or the composition as claimed in claim 16, for use as a medicament.

21. The pharmaceutical composition as claimed in claim 18, for use in treating diseases caused by coronaviruses or symptoms thereof; preferably, the coronavirus is selected from SARS-CoV-2, SARS-CoV and MERS-CoV.

22. A method for treating diseases caused by coronaviruses or symptoms thereof in a subject, comprising the step of administering a therapeutically effective amount of the crystal form as claimed in any of claims 1-16 to the subject; preferably, the disease is selected from COVID-19, SARS and MERS.
